Europäisches Patentamt

European Patent Office

Office européen des brevets

(19)

(11) Publication number : **0 500 290 A2**

# EUROPEAN PATENT APPLICATION

(12)

(21) Application number : 92301266.0

(51) Int. Cl.⁵ : **C12Q 1/68**

(22) Date of filing : 17.02.92

The applicant has subsequently filed a sequence listing and declared, that it includes no new matter.

(30) Priority : 22.02.91 US 659974
15.03.91 US 670242

(43) Date of publication of application :
26.08.92 Bulletin 92/35

(84) Designated Contracting States :
AT BE CH DE DK ES FR GB GR IT LI LU MC NL PT SE

(71) Applicant : **THE REGENTS OF THE UNIVERSITY OF CALIFORNIA**
**300 Lakeside Drive**
**Oakland, California 94501 (US)**

(72) Inventor : **Gray, Joe W.**
**1760 Lomitas Avenue**
**Livermore, California 94550 (US)**
Inventor : **Pinkel, Daniel**
**31 Manzanita Court**
**Walnut Creek, California 94595 (US)**
Inventor : **Kallioniemi, Olli-Pekka**
**Laljankuja 4**
**SF-333-00 Tampere (FI)**
Inventor : **Kallioniemi, Anne**
**Laljankuja 4**
**SF-333-00 Tampere (FI)**
Inventor : **Sakamoto, Masaru**
**4-1-10 Nishishinjuku No.207, Sunjuku**
**Tokyo (JP)**

(74) Representative : **Harrison, David Christopher et al**
**MEWBURN ELLIS 2 Cursitor Street**
**London EC4A 1BQ (GB)**

(54) Chromosome-specific staining to detect genetic rearrangements.

(57) Provided are methods and compositions for staining chromosomal material based upon nucleic acid sequence employing high complexity nucleic acid probes wherein the staining pattern produced is indicative of the presence or absence of one or more genetic rearrangements involving chromosomes 3, 13 and/or 17. Said probes are appropriate for in situ hybridization and stain both interphase and metaphase chromosomal material with reliable signals. The staining methods can be used in conjunction with digital image analysis. Probes and test kits are provided for use in detecting genetic rearrangements, particularly those related to tumor suppressor genes, in the detection of disease related loci, specifically cancer, such as retinoblastomas, osteosarcomas, lung cancer, breast cancer, ovarian and uterine cancers, and for biological dosimetry. Reference probes, for example, centromeric alpha satellite probes, can be a component of the high complexity probes.

EP 0 500 290 A2

The United States Government has rights in this invention pursuant to Contract No. W-7405-ENG-48 between the U.S. Department of Energy and the University of California, for the operation of Lawrence Livermore National Laboratory.

## FIELD OF THE INVENTION

The invention relates generally to the field of cytogenetics, and more particularly, to the field of molecular cytogenetics. The invention concerns methods for identifying and classifying chromosomes. Still more particularly, this invention concerns high complexity nucleic acid probes which can be designed by the processes described herein to produce staining distributions that can extend along one or more whole chromosomes, and/or along a region or regions on one or more chromosomes, including staining patterns that extend over the whole genome. Staining patterns can be tailored for any desired cytogenetic application, including prenatal, tumor and disease related cytogenetic applications, among others. The invention provides for compositions of nucleic acid probes and for methods of staining chromosomes therewith to identify normal chromosomes and chromosomal abnormalities in metaphase spreads and in interphase nuclei. The probe-produced staining patterns of this invention facilitate the microscopic and/or flow cytometric identification of normal and abnormal chromosomes and the characterization of the genetic nature of particular abnormalities.

Although most of the examples herein concern human chromosomes and much of the language herein is directed to human concerns, the concept of using nucleic acid probes for staining or painting chromosomes is applicable to chromosomes from any source including both plants and animals.

## BACKGROUND OF THE INVENTION

Chromosome abnormalities are associated with genetic disorders, degenerative diseases, and exposure to agents known to cause degenerative diseases, particularly cancer, German, "Studying Human Chromosomes Today," American Scientist, Vol. 58, pgs. 182-201 (1970); Yunis, "The Chromosomal Basis of Human Neoplasia," Science, Vol. 221, pgs. 227-236 (1983); and German, "Clinical Implication of Chromosome Breakage," in Genetic Damage in Man Caused by Environmental Agents, Berg, Ed., pgs. 65-86 (Academic Press, New York, 1979). Chromosomal abnormalities can be of several types, including: extra or missing individual chromosomes, extra or missing portions of a chromosome (segmental duplications or deletions), breaks, rings and chromosomal rearrangements, among others. Chromosomal or genetic rearrangements include translocations (transfer of a piece from one chromosome onto another chromosome), dicentrics (chromosomes with two centromeres), inversions (reversal in polarity of a chromosomal segment), insertions, amplifications, and deletions.

Detectable chromosomal abnormalities occur with a frequency of one in every 250 human births. Abnormalities that involve deletions or additions of chromosomal material alter the gene balance of an organism and generally lead to fetal death or to serious mental and physical defects. Down syndrome can be caused by having three copies of chromosome 21 instead of the normal 2. This syndrome is an example of a condition caused by abnormal chromosome number, or aneuploidy. Down syndrome can also be caused by a segmental duplication of a subregion on chromosome 21 (such as, 21q22), which can be present on chromosome 21 or on another chromosome. Edward syndrome (18+), Patau syndrome (13+), Turner syndrome (XO) and Kleinfelter syndrome (XXY) are among the most common numerical aberrations. [Epstein, The Consequences of Chromosome Imbalance: Principles, Mechanisms and Models (Cambridge Univ. Press 1986); Jacobs, Am. J. Epidemiol, 105:180 (1977); and Lubs et al., Science, 169:495 (1970).]

Retinoblastoma (del 13q14), Prader-Willi syndrome (del 15q11- q13), Wilm's tumor (del 11p13) and Cri-du-chat syndrome (del 5p) are examples of important disease linked structural aberrations. [Nora and Fraser, Medical Genetics: Principles and Practice, (Lea and Febiger 1989).]

Manuelidis et al., "Chromosomal and Nuclear Distribution of the Hind III 1.9-KB Human DNA Repeat Segment," Chromosoma, 91: 28-38 (1984), disclose the construction of a single kind of DNA probe for detecting multiple loci on chromosomes corresponding to the location of members of a family of repeated DNA sequences. Such probes are herein termed repetitive probes.

Different repetitive sequences may have different distributions on chromosomes. They may be spread over all chromosomes as in the just cited reference, or they may be concentrated in compact regions of the genome, such as, on the centromeres of the chromosomes, or they may have other distributions. In some cases, such a repetitive sequence is predominantly located on a single chromosome, and therefore is a chromosome-specific repetitive sequence. [Willard et al., "Isolation and Characterization of a Major Tandem Repeat Family from the Human X Chromosome," Nuc. Acids Research, 11: 2017-2033 (1983).]

Recently, there has been an increased availability of probes for repeated sequences (repetitive probes) that hybridize intensely and specifically to selected chromosomes. [Trask et al., Hum. Genet., 78:251 (1988) and references cited therein.] Such probes are now available for over half of the human chromosomes. In general, they bind to repeated sequences on compact regions of the target chromosome near the centromere. However, one probe has been reported that hybridizes to human chromosome 1p36, and there are several probes that hybridize to human chromosome Yq. Hybridization with such probes permits rapid identification of chromosomes in metaphase spreads, determination of the number of copies of selected chromosomes in interphase nuclei [Pinkel et al. (I), PNAS USA, 83:2934 (1986); Pinkel et al. (II), Cold Spring Harbor Symp. Quant. Biol., 51:151 (1986) and Cremer et al., Hum. Genet, 74:346 (1986)] and determination of the relative positions of chromosomes in interphase nuclei [Trask et al., Supra; Pinkel et al. (I), supra; Pinkel et al. (II), supra; Manuelidis, PNAS USA, 81:3123 (1984); Rappold et al., Hum. Genet., 67:317 (1984); Schardin et al., Hum. Genet., 71:282 (1985); and Manuelidis, Hum. Genet., 71:288 (1985)]. However, repetitive probes are not very useful for detection of structural aberrations since the probability is low that the aberrations will involve the region to which the probe hybridizes.

Exemplified herein are probes in an approximate complexity range of from about 50,000 bases (50 kb) to hundreds of millions of bases. Such representative probes are for compact loci and whole human chromosomes.

The techniques of this invention can be especially advantageous for applications where high-quality banding by conventional methods is difficult or suspected of yielding biased information, e.g., in tumor cytogenetics. Reagents targeted to sites of lesions known to be diagnostically or prognostically important, such as tumor type-specific translocations and deletions, among other tumor specific genetic arrangements, permit rapid recognition of such abnormalities. Where speed of analysis is the predominant concern, e.g., detection of low-frequency chromosomal aberrations induced by toxic environmental agents, the compositions of this invention permit a dramatic increase in detection efficiency in comparison to previous techniques based on conventional chromosome banding.

Further, prenatal screening for disease-linked chromosome aberrations (e.g., trisomy 21) is enhanced by the rapid detection of such aberrations by the methods and compositions of this invention. Interphase aneuploidy analysis according to this invention is particularly significant for prenatal diagnosis in that it yields more rapid results than are available by cell culture methods. Further, fetal cells separated from maternal blood, which cannot be cultured by routine procedures and therefore cannot be analysed by conventional karyotyping techniques, can be examined by the methods and compositions of this invention. In addition, the intensity, contrast and color combinations of the staining patterns, coupled with the ability to tailor the patterns for particular applications, enhance the opportunities for automated cytogenetic analysis, for example, by flow cytometry or computerized microscopy and image analysis.

This application specifically claims chromosome specific reagents for the detection of genetic rearrangements and methods of using such reagents to detect such rearrangements. Representative genetic rearrangements so detected are those associated with chromosomes 3, 13 (retinoblastoma tumor suppressor gene therein), and 17, such as deletions, amplifications including aneuploidy and translocations thereof.

Chromosomal deletions involving tumor suppressor genes may play an important role in the development and progression of solid tumors. The retinoblastoma tumor suppressor gene (Rb-1), located in chromosome 13q14, is the most extensively characterized tumor suppressor gene [Friend et al., Nature, 323: 643 (1986); Lee et al., Science, 235: 1394 (1987); Fung et al., Science, 236: 1657 (1987)]. The Rb-1 gene product, a 105 kDa nuclear phosphoprotein, apparently plays an important role in cell cycle regulation [Lee et al., supra (1987); Howe et al., PNAS (USA), 87: 5883 (1990)]. Altered or lost expression of the Rb protein is caused by inactivation of both gene alleles either through a point mutation or a chromosomal deletion. Rb-1 gene alterations have been found not only in retinoblastomas [Friend et al., Supra (1986); Lee et al., supra (1987); Fung et al., supra (1987)] but also in other malignancies such as osteosarcomas [Friend et al., supra (1986)], small cell lung cancer [Hensel et al., Cancer Res., 50: 3067 (1990); Rygaard et al., Cancer Res., 50: 5312 (1990)] and breast cancer [Lee et al., Science, 241:218 (1988); T'Ang et al., Science, 242: 263 (1988); Varley et al., Oncogene, 4: 725 (1989)]. Restriction fragment length polymorphism (RFLP) studies have indicated that such tumor types have frequently lost heterozygosity at 13q suggesting that one of the Rb-1 gene alleles has been lost due to a gross chromosomal deletion [Bowcock et al., Am. J. Hum. Genet., 46: 12 (1990)].

Section VI infra describes the use of fourteen lambda phage clones spanning all the exons of the Rb-1 gene region, about 150 kb of genomic DNA, as a high complexity probe for chromosome-specific painting. An intense signal produced in metaphase chromosomes confirmed the location of the Rb-1 gene at chromosome 13q14. Two Rb-1 hybridization signals were detected in about 90% of normal interphase nuclei, whereas two cell lines having a cytogenetically defined deletion involving the Rb-1 gene region showed only one hybridization signal. Gene deletion was confirmed by analyzing metaphase spreads from these cell lines cohybridized with chromosome 13/21 alpha satellite probe. Also analyzed were touch preparations and fine needle aspirates of breast carcinomas; heterogeneity was shown in Rb-1 gene copy number both within and between tumors.

Genetic rearrangements involving only subregions of the Rb-1 gene have been described [Bookstein et al., PNAS (USA) 85: 2210 (1988); Canning and Dryja, PNAS, 86: 5044 (1989). To detect subregions of the Rb-1 gene by the methods of this invention, smaller probes comprising 1-5 contiguous lambda phage clones were used to stain specific subregions within the Rb-1 gene thus allowing detection of aberrations within that tumor suppressor gene. Such representative examples of the chromosome-specific staining methods of this invention provide information on actual gene copy numbers and rearrangements from individual morphologically defined tumor cells useful in the evaluation of neoplasia-associated gene aberrations as well as intratumor genetic heterogeneity.

The deletion of the short arm of chromosome 3 has been associated with several cancers, for example, small cell lung cancer, renal and ovarian cancers; it has been postulated that one or more putative tumor suppressor genes is or are located in th p region of chromosome 3 (ch. 3p) [Minna et al., Symposia on Quantitative Biology, Vol. L1:843-853 (SCH Lab 1986); Cohen et al., N. Eng. J. Med., 301:592-595 (1979); Bergerham et al., Cancer Res., 49:1390-1396 (1989); Whang-Peng et al., Can. Genet. Cytogenet., 11:91-106 (1984); and Trent et al., Can. Genet. Cytogenet., 14:153161 (1985)]. As shown in Section VII infra, chromosome-specific staining according to this invention can be used to create bands of stained nucleic acid that detect structural aberrations, for example, those of chromosome 3.

Described herein are chromosome-specific reagents and methods to detect genetic rearrangements, such as those exemplified deletions, amplifications, and translocations of chromosomes 3, 17 and 13.

SUMMARY OF THE INVENTION

This invention concerns methods of staining chromosomal material based upon nucleic acid sequence employing high complexity nucleic acid probes wherein the staining pattern produced is indicative of the presence or absence of one or more genetic rearrangements. The exemplary genetic rearrangements herein disclosed are those associated with the retinoblastoma turmor suppressor gene on chromosome 13 and those associated with chromosomes 3 and 17.

The invention provides compositions for staining the targeted chromosomal material. The probe compositions of this invention at the current state of hybridization techniques are typically of high complexity, usually greater than about 50 kb of complexity, the complexity depending upon the application for which the probe is designed.

This invention concerns chromosome specific reagents and methods of staining targeted chromosomal material that is in the vicinity of one or more suspected genetic rearrangements. Such genetic rearrangements include but are not limited to translocations, inversions, insertions, amplifications and deletions. Aneuploidy is included herein in the term "amplifications".

The presence of a genetic rearrangement can be determined by applying the reagents of this invention according to methods herein described and observing the proximity of and/or other characteristics of the signals of the staining patterns produced.

The invention provides methods of staining the targeted chromosomal material whether it is in metaphase spreads or interphase nuclei. It is a particular object of this invention to stain cells from clinical specimens. A particular subject of this invention is chromosomal material from cells that are suspected of being cancerous. Of particular interest according to the instantly disclosed invention, are cancers associated with chromosomal deletions involving tumor suppressor genes, notably the retinoblastoma tumor suppressor gene (Rb-1) located on chromosome 13, and the tumor suppressor genes on the p arm of chromosome 3; such cancers are selected from the group consisting of retinoblastomas, osteosarcomas, lung cancer (particularly, small cell lung cancer), breast cancer, renal cell cancer, ovarian cancer and uterine cancer. Particularly associated with genetic rearrangements, notably deletions, in the Rb-1 gene are retinoblastomas, osteosarcomas, small cell lung cancer and breast cancer. Particularly associated with genetic rearrangements, notably deletions, in the p arm of chromosome 3 are small cell lung cancer, renal cell cancer, uterine and/or ovarian cancers.

Thus, the invention provides for high complexity nucleic acid probes for the detection of one or more genetic rearrangements associated with the retinoblastoma tumor suppressor gene (Rb-1), chromosome 3, and/or chromosome 17. The methods using such probes can be considered diagnostic and/or prognostic of cancer.

The methods of this invention can comprise the use of reference probes, for example, centromeric-specific alpha satellite probes, such as, a chromosome 13/21 centromeric probe, or chromosome 3 centromeric probe and/or a chromosome 17 centromeric probe.

One way to produce a probe of high complexity is to pool several or many clones, for example, phage, plasmid, cosmid, and/or YAC clones, among others, wherein each clone contains an insert that is capable of hybridizing to some part of the target in a genome. Cosmids are a preferred vector for locus-specific probes of this invention. Another way to produce such a probe is to use the polymerase chain reaction (PCR), for example, by the PCR adapter-linker method.

Heterogeneous in reference to the mixture of labeled nucleic acid fragments means that the staining reagents comprise many copies each of fragments having different sequences and/or sizes (e.g., from the different DNA clones pooled to make the probe). In preparation for use, these fragments may be cut, randomly or specifically, to adjust the size distribution of the pieces of nucleic acid participating in the hybridization reaction.

As discussed more fully below, preferably the heterogeneous probe mixtures are substantially free from nucleic acid sequences with hybridization capacity to non-targeted nucleic acid. Most of such sequences bind to repetitive sequences which are shared by the target and non-target nucleic acids, that is, shared repetitive sequences. Methods to remove undesirable nucleic acid sequences and/or to disable the hybridization capacity of such sequences are discussed below in Section II.

A preferred method of making the chromosome-specific staining reagents of the invention includes: 1) isolating chromosomal DNA from a particular chromosome type or target region or regions in the genome, 2) amplifying the isolated DNA to form a heterogeneous mixture of nucleic acid fragments, 3) disabling the hybridization capacity of or removing shared repeated sequences in the nucleic acid fragments, and 4) labeling the nucleic acid fragments to form a heterogeneous mixture of labeled nucleic acid fragments. As described more fully below, the ordering of the steps for particular embodiments varies according to the particular means adopted for carrying out the steps.

Disclosed herein are methods of detecting genetic rearrangements associated with the retinoblastoma tumor suppressor gene, chromosome 3, and/or chromosome 17 in humans comprising the steps of:

a. hybridizing appropriate high complexity nucleic acid probes, as exemplified herein, to targeted chromosomal material;

b. observing and/or measuring the proximity of and/or other characteristics of the signals from said probes; and

c; determining from said observations and/or measurements obtained in step b) whether one or more genetic rearrangements has or have occurred.

Further disclosed herein are chromosome-specific staining reagents that provide staining patterns indicative of one or more genetic rearrangements associated with the retinoblastoma tumor suppressor gene, chromosome 3, and/or chromosome 17 in humans, produced by the process of:

isolating chromosome-specific DNA selected from the group consisting of chromosomes 3, 13 and 17;

amplifying pieces of the isolated chromosome-specific DNA;

disabling the hybridization capacity of and/or removing shared repetitive sequences contained in the amplified pieces of the isolated DNA to form a collection of nucleic acid fragments which hybridize predominantly to targeted chromosomal DNA on chromosomes 3, 13 and/or 17; and

labeling the nucleic acid fragments of the collection to form a heterogeneous mixture of nucleic acid fragments. The amplification step can be achieved, for example, by cloning and/or by using the polymerase chain reaction (PCR), including the PCR adapter-linker method.

Digital image analysis is disclosed herein as a useful adjunct to the staining methods of this invention, performed in conjunction therewith.

The methods of this invention include staining targeted chromosomal material based upon nucleic acid sequence employing high complexity nucleic acid probes wherein the staining pattern produced is indicative of one or more genetic rearrangements associated with a tumor suppressor gene. A representative method is that wherein the tumor suppressor gene is either the retinoblastoma tumor suppressor gene or located on the p arm of chromosome 3, and wherein the genetic rearrangement is a deletion.

This invention still further provides for test kits comprising appropriate nucleic acid probes for use in tumor cytogenetics, in the detection of disease related loci, in the analysis of structural abnormalities, for example translocations, among other genetic rearrangements, and for biological dosimetry.

This invention further provides for prenatal screening kits comprising appropriate nucleic acid probes of this invention.

It is further an object of the instant invention to detect small specific deletions invisible by conventional banding analyses, including subregion deletions within a gene, for example, the Rb-1 gene.

It is still further an object of this invention to provide methods of studying the genetic heterogeneity of cancers, such as, solid tumors, and of gene copy numbers and structural abnormalities from morphologically defined individual tumor cells.

The invention further provides for automated means of detecting and analyzing chromosomal abnormalities, particularly genetic rearrangements, as indicated by the staining patterns produced according to this invention.

## BRIEF DESCRIPTION OF THE DRAWINGS

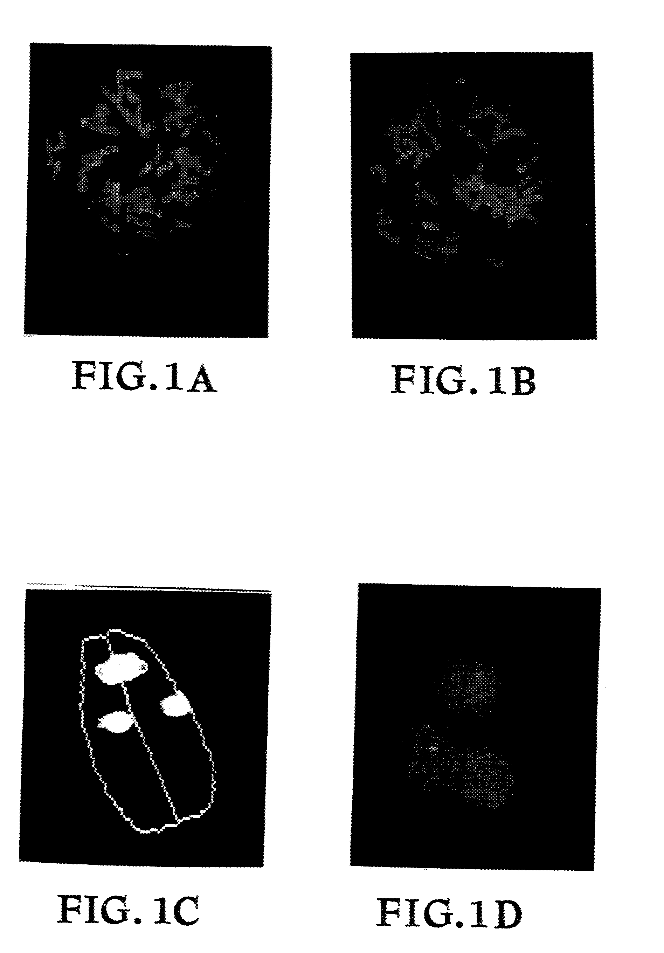

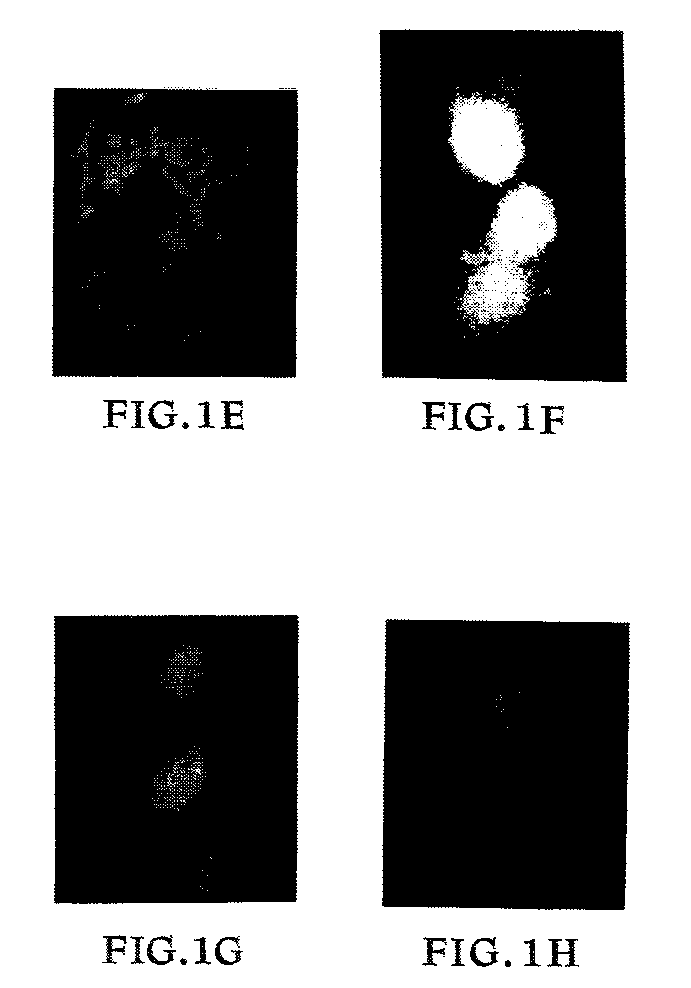

Figure 1 shows fluoresence in situ hybridization (FISH) with fourteen Rb-1 lambda phage clones (Rb-1 probe) in normal and abnormal metaphase spreads and interphase nuclei. Panels A and B show two pairs of bright and specific hybridization signals on normal lymphocyte metaphase preparations in the mid-region of the q-arm of chromosome 13. Panel B further shows cohybridization with a 13/21 centromeric probe. Panel C shows a digital image analysis of the mapping of the Rb-1 gene on a metaphase chromosome using both the Rb-1 probe and the 13/21 centromere specific repeat probe. Panel D shows two bright and specific hybridization domains in interphase nuclei of normal lymphocytes. Panel E shows cohybridization of the Rb-1 probe and a 13/21 centromere specific repeat probe to metaphase spreads of a fibroblast cell line (GMO5877) derived from a sporadic retinoblastoma patient. Intact chromosome 13s show both Rb-1 and centromere signals; whereas chromosome 13s with a Rb-1 deletion are slightly shortened and hybridize only with the centromeric probe. Panel F shows a digital image analysis of the GM05887 cell line metaphase showing both the normal and shortened chromosome 13 and wherein cohybridization was effected with both the Rb-1 and 13/21 centromeric probe. Panel G shows hybridization of the Rb-1 probe to a GM05887 cell line interphase. Panel H shows hybridization of the Rb-1 probe to a clinical breast cancer specimen. Panel I shows a digital image analysis of a dual color hybridization to a normal interphase nucleus; differently labeled portions of the Rb-1 probe--a 3'-(green) portion and a 5' (red) portion--were hybridized to the normal interphase nucleus.

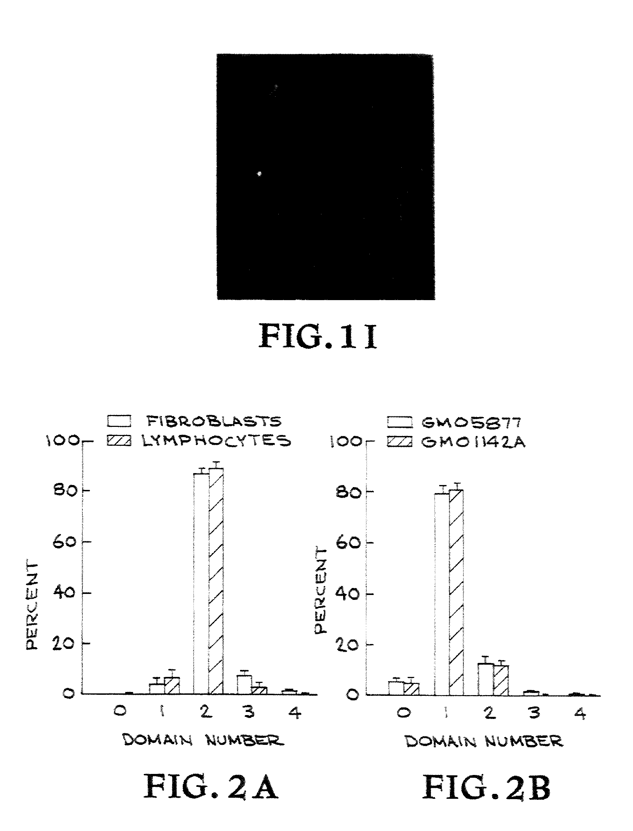

Figure 2 graphically illustrates the distribution of Rb-1 gene hybridization signals in (A) interphase nuclei of normal peripheral blood lymphocytes and fibroblasts; and in (B) two cell lines with a cytogenetically defined deletion involving the Rb-1 gene at 13q. The results represent the mean (+S.D.) of 3-5 different hybridization experiments. At least 150 nuclei were scored from each slide.

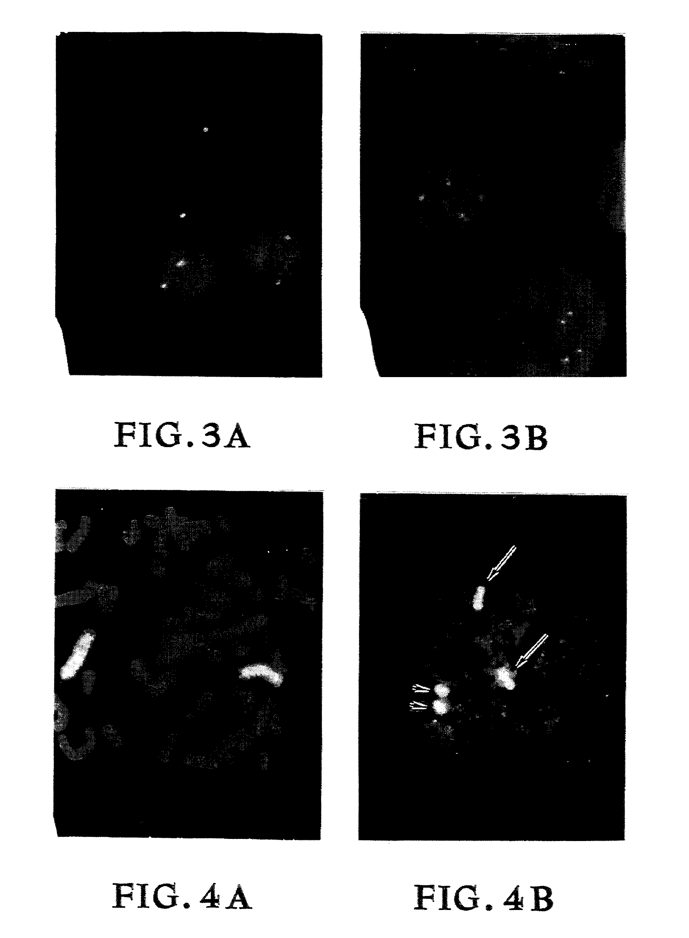

Figure 3 shows FISH with chromosome 17 centromeric-specific alpha satellite probe. Panel A shows such hybridization to normal lymphocytes wherein in metaphase chromosomes, two chromosome 17 centromeric-specific bright signals are seen, and in interphase nuclei, corresponding bright and tight hybridization domains are visible. Panel B shows such hybridization to an ovarian cancer cell line (RMUG-L) wherein in both metaphase and interphase, four signals are visible, indicating aneuploidy of chromosome 17.

Figure 4 shows FISH with a whole chromosome composite probe for chromosome 3. Panel A shows such a hybridization to normal lymphocytes wherein the whole body of chromosome 3 was homogeneously painted with fluorescein isothiocyanate (FITC). Panel B shows such a hybridization to an ovarian cancer cell line (RMUG-L) wherein four chromosome 3s are specifically stained with FITC and wherein two (to which short arrows point) out of the four are apparently shorter than the intact chromosome 3s (to which long arrows point). The shorter chromosomes are considered to correspond to those with a 3p deletion.

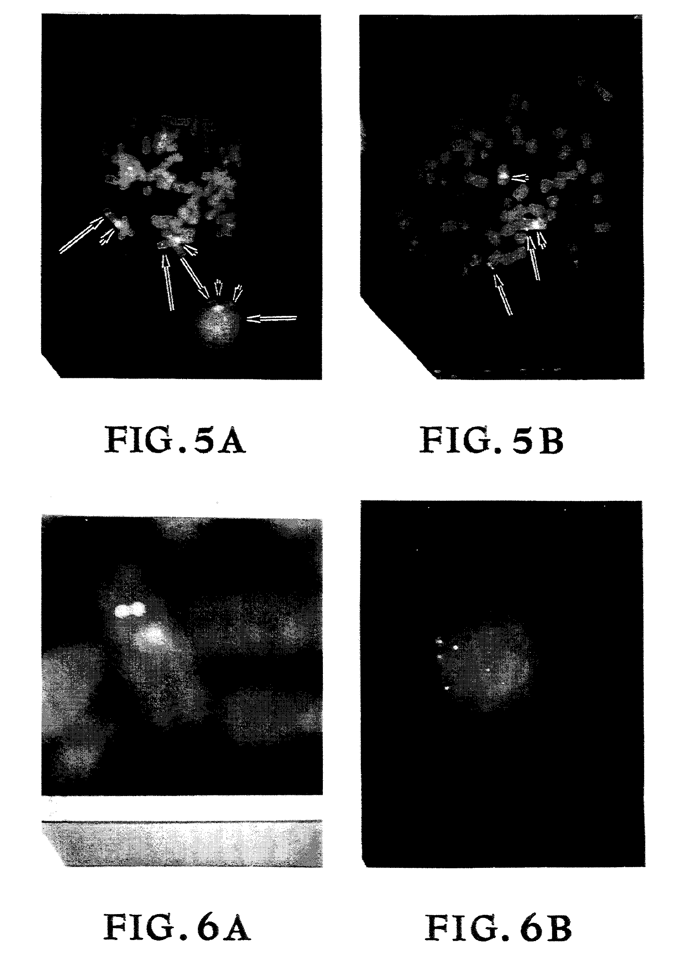

Figure 5 shows simultaneous hybridization with a chromosome 3 centromeric-specific probe generated by the polymerase chain reaction (PCR) and a chromosome 3 locus-specific cosmid probe (mapped to ch. 3q26 by digital image analysis). Panel A shows such a hybridization to metaphase spreads and interphase nuclei from normal lymphocytes wherein two chromosome 3 centromeric-specific signals (indicated by short arrows) and two pairs of chromosome 3q cosmid signals (indicated by long arrows) are clearly visible in the metaphase spreads; and wherein two large hybridization domains for the chromosome 3 centromeres and two small domains for the chromosome 3q locus-specific probes are visible in the interphase nuclei. Panel B shows such a hybridization to a uterine cervical adenocarcinoma cell line (TMCC-1) wherein two chromsome 3 centromeric-specific (indicated by short arrows) and two chromosome 3q locus-specific cosmid (indicated by long arrows) signals are clearly visible in metaphase spreads whereas a pair of cosmid signals specific to chromosome 3q are found to be translocated to another chromosome.

Figure 6 shows the simultaneous dual color hybridization with a chromosome 3 centromeric-specific probe (green) and a chromsome 3 locus-specific cosmid probe mapped to 3p21 (red) to (A) metaphase spreads and (B) interphase nuclei of normal lymphocytes.

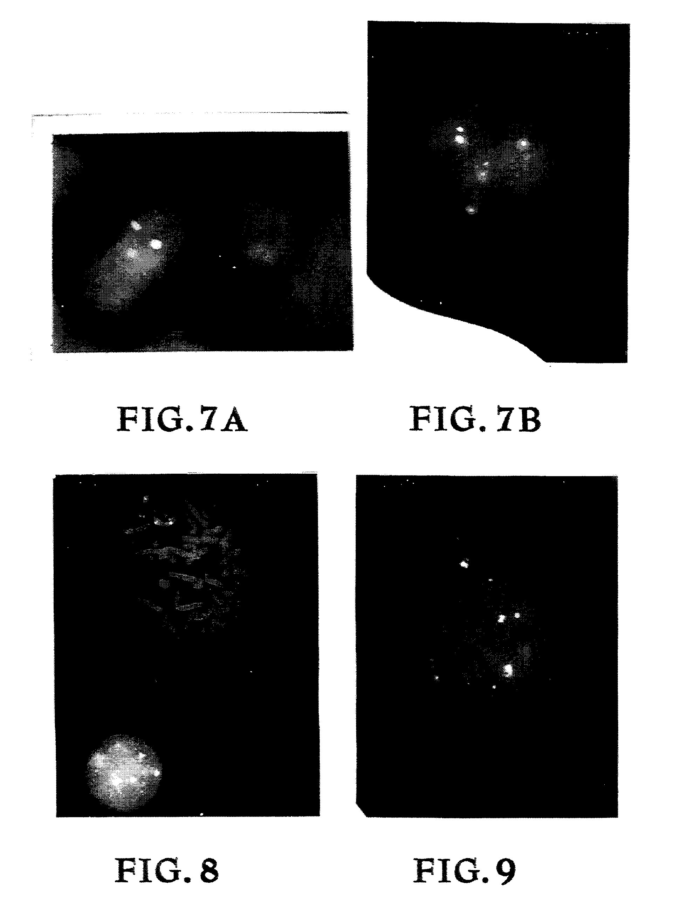

Figure 7 shows hybridizations corresponding to those shown in Figure 6 wherein the metaphase spreads (A) and interphase nucleus (B) are from an ovarian cancer cell line (RMUG-S). In Panel A, the metaphase chromosome on the right exhibits an apparent 3p deletion whereas the metaphase chromosome on the left appears intact. In Panel B, chromosome 3 aneuploidy is demonstrated by the four green centromeric domains; two intact chromosome 3s are indicated by two pairs of adjacent green and red dots; and two 3p deleted chromosome 3s are indicated by the two single green domains.

Figure 8 shows the simultaneous hybridizations of AAF-labeled chromosome 3 centromeric-specific probe (from H. Willard at Stanford) and a biotinylated chromosome 3q cosmid probe (J14R1A12; probes described infra under Section VII) to a metaphase spread and interphase nucleus of normal lymphocytes. A normal pattern is shown, that is, two green and two pairs of red signals per cell.

Figure 9 shows hybridizations comparable to that shown in Figure 8 except that the interphase nucleus is from an ovarian cancer cell line (RMUG-S). An abnormal pattern is shown, that is, four chromosome 3 centromeric-specific green signals and four chromosome 3q cosmid red signals, indicating that the nucleus contains four long arms of chromosome 3.

DETAILED DESCRIPTION OF THE INVENTION

This invention concerns the use of nucleic acid probes to stain targeted chromosomal material in patterns which can extend along one or more whole chromosomes, and/or along one or more regions on one or more chromosomes, including patterns which extend over an entire genome. The staining reagents of this invention facilitate the microscopic and/or flow cytometric identification of normal and aberrant chromosomes and provide for the characterization of the genetic nature of particular abnormalities, such as, genetic rearrangements. The term "chromosome-specific" is herein defined to encompass the terms "target specific" and "region specific", that is, when the staining composition is directed to one chromosome, it is chromosome-specific, but it is also chromosome-specific when it is directed, for example, to multiple regions on multiple chromosomes, or to a region of only one chromosome, or to regions across the entire genome. The term chromosome-specific originated from the use of recombinant DNA libraries made by cloning DNA from a single normal chromosome type as the source material for the initial probes of this invention. Libraries made from DNA from regions of one or more chromosomes are sources of DNA for probes for that region or those regions of the genome. The probes produced from such source material are region-specific probes but are also encompassed within the broader phrase "chromosome-specific" probes. The term "target specific" is interchangeably used herein with the term "chromosome-specific".

The word "specific" as commonly used in the art has two somewhat different meanings. The practice is followed herein. "Specific" may refer to the origin of a nucleic acid sequence or to the pattern with which it will hybridize to a genome as part of a staining reagent. For example, isolation and cloning of DNA from a specified chromosome results in a "chromosome-specific library". [Eg., Van Dilla et al., "Human Chromosome-Specific DNA Libraries: Construction and Availability," Biotechnology, 4:537 (1986).] However, such a library contains sequences that are shared with other chromosomes. Such shared sequences are not chromosome-specific to the chromosome from which they were derived in their hybridization properties since they will bind to more than the chromosome of origin. A sequence is "chromosome-specific" if it binds only to the desired portion of a genome. Such sequences include single-copy sequences contained in the target or repetitive sequences, in which the copies are contained predominantly in the target.

"Chromosome-specific" in modifying "staining reagent" refers to the overall hybridization pattern of the nucleic acid sequences that comprise the reagent. A staining reagent is chromosome-specific if useful contrast between the target and non-target chromosomal material is achieved (that is, that the target can be adequately visualized).

A probe is herein defined to be a collection of nucleic acid fragments whose hybridization to the target can be detected. The probe is labeled as described below so that its binding to the target can be visualized. The probe is produced from some source of nucleic acid sequences, for example, a collection of clones or a collection of polymerase chain reaction (PCR) products. The source nucleic acid may then be processed in some way, for example, by removal of repetitive sequences or blocking them with unlabeled nucleic acid with complementary sequence, so that hybridization with the resulting probe produces staining of sufficient contrast on the target. Thus, the word probe may be used herein to refer not only to the detectable nucleic acid, but also to the detectable nucleic acid in the form in which it is applied to the target, for example, with the blocking nucleic acid, etc. The blocking nucleic acid may also be mentioned separately. What "probe" refers to specifically should be clear from the context in which the word is used.

When two or more nucleic acid probes of this invention are mixed together, they produce a new probe which when hybridized to a target according to the methods of this invention, produces a staining pattern that is a combination of the staining patterns individually produced by the component probes thereof. Thus, the terms "probe" and "probes" (that is, the singular and plural forms) can be used interchangeably within the context of a staining pattern produced. For example, if one probe of this invention produces a dot on chromosome 9, and another probe produces a band on chromosome 11, together the two probes form a probe which produces a dot/band staining pattern.

The term "labeled" is herein used to indicate that there is some method to visualize the bound probe, whether or not the probe directly carries some modified constituent. Section III infra describes various means of directly labeling the probe and other labeling means by which the bound probe can be detected.

The terms "staining" or "painting" are herein defined to mean hybridizing a probe of this invention to a genome or segment thereof, such that the probe reliably binds to the targeted chromosomal material therein and the bound probe is capable of being visualized. The terms "staining" or "painting" are used interchangeably.

The patterns resulting from "staining" or "painting" are useful for cytogenetic analysis, more particularly, molecular cytogenetic analysis. The staining patterns facilitate the microscopic and/or flow cytometric identification of normal and abnormal chromosomes and the characterization of the genetic nature of particular abnormalities. Section III infra describes methods of rendering the probe visible. Since multiple compatible methods of probe visualization are available, the binding patterns of different components of the probe can be distinguished--for example, by color. Thus, this invention is capable of producing any desired staining pattern on the chromosomes visualized with one or more colors (a multi-color staining pattern) and/or other indicator methods. The term "staining" as defined herein does not include the concept of staining chromosomes with chemicals as in conventional karotyping methods although such conventional stains may be used in conjunction with the probes of this invention to allow visualization of those parts of the genome where the probe does not bind. The use of DAPI and propidium iodide for such a purpose is illustrated in the figures.

The phrase "high complexity" is defined herein to mean that the probe, thereby modified contains on the order of 50,000 (50 kb) or greater, up to many millions or several billions, of bases of nucleic acid sequences which are not repeated in the probe. For example, representative high complexity nucleic acid probes of this invention can have a complexity greater than 50 kb, greater than 100,000 bases (100 kb), greater than 200,000 (200 kb), greater than 500,000 bases (500 kb), greater than one million bases (1 Mb), greater than 2 Mb, greater than 10 Mb, greater than 100 Mb, greater than 500 Mb, greater than 1 billion bases and still further greater than several billion bases.

The term "complexity" is defined herein according to the standard for nucleic acid complexity as established by Britten et al., Methods of Enzymol., 29:363 (1974). See also Cantor and Schimmel, Biophysical Chemistry: Part III: The Behavior of Biological Macromolecules, at 1228-1230 (Freeman and Co. 1980) for further explanation and exemplification of nucleic acid complexity.

The complexity preferred for a probe composition of this invention is dependent upon the application for which it is designed. In general, the larger the target area, the more complex is the probe. It is anticipated that the complexity of a probe needed to produce a desired pattern of landmarks on a chromosome will decrease as hybridization sensitivity increases, as progress is made in hybridization technology. As the sensitivity increases, the reliability of the signal from smaller target sites will increase. Therefore, whereas from about a 40 kb to about a 100 kb target sequence may be presently necessary to provide a reliable, easily detectable signal, smaller target sequences should provide reliable signals in the future. Therefore, as hybridization sensitivity increases, a probe of a certain complexity, for example, 100 kb, should enable the user to detect considerably more loci in a genome than are presently reliably detected; thus, more information will be obtained with a probe of the same complexity. The term "complexity" therefore refers to the complexity of the total probe no matter how many visually distinct loci are to be detected, that is, regardless of the distribution of the target sites over the genome.

As indicated above, with current hybridization techniques it is possible to obtain a reliable, easily detectable signal with a probe of about 40 kb to about 100 kb (eg. the probe insert capacity of one or a few cosmids) targeted to a compact point in the genome. Thus, for example, a complexity in the range of approximately 100 kb now permits hybridization to both sides of a tumor-specific translocation. The portion of the probe targeted to one side of the breakpoint can be labeled differently from that targeted to the other side of the breakpoint so that the two sides can be differentiated with different colors, for example. Proportionately increasing the complexity of the probe permits analysis of multiple compact regions of the genome simultaneously. The conventional banding patterns produced by chemical stains may be replaced according to this invention with a series of probe-based, color coded (for example), reference points along each chromosome or significant regions thereof.

Uniform staining of an extended contiguous region of a genome, for example, a whole chromosome, requires a probe complexity proportional to but substantially less than, the complexity of the target region. The complexity required is only that necessary to provide a reliable, substantially uniform signal on the target.

Increasing the complexity beyond the minimum required for adequate staining is not detrimental as long as the total nucleic acid concentration in the probe remains below the point where hybridization is impaired. The decrease in concentration of a portion of a sequence in the probe is compensated for by the increase in the number of target sites. In fact, when using double-stranded probes, it is preferred to maintain a relatively low concentration of each portion of sequence to inhibit reassociation before said portion of sequence can find a binding site in the target.

The staining patterns of this invention comprise one or more "bands". The term "band" is herein defined as a reference point in a genome which comprises a target nucleic acid sequence bound to a probe component, which duplex is detectable by some indicator means, and which at its narrowest dimension provides for a reliable signal under the conditions and protocols of the hybridization and the instrumentation, among other variables, used. A band can extend from the narrow dimension of a sequence providing a reliable signal to a whole chromosome to multiple regions on a number of chromosomes.

The probe-produced bands of this invention are to be distinguished from bands produced by chemical staining as indicated above in the Background. The probe-produced bands of this invention are based upon nucleic acid sequence whereas the bands produced by chemical staining depend on natural characteristics of the chromosomes, but not the actual nucleic acid sequence. Further, the banding patterns produced by chemical staining are only interpretable in terms of metaphase chromosomes whereas the probe-produced bands of this invention are useful both for metaphase and interphase chromosomes.

One method of forming the probes of the present invention is to pool many different low complexity probes. Such a probe would then comprise a "heterogeneous mixture" of individual cloned sequences. The number of clones required depends on the extent of the target area and the capacity of the cloning vector. If the target is made up of several discrete, compact loci, that is, single spots at the limit of microscopic resolution, then about 40 kb, more preferably 100 kb, for each spot gives a reliable signal given current techniques. The portion of the probe for each spot may be made up from, for example, a single insert from a yeast artificial chromosome (YAC), from several cosmids each containing 35-40 kb or probe sequence, or from about 25 plasmids each with 4 kb of sequence.

Representative heterogeneous mixtures of clones of this invention include but are not limited to phage, plasmids, cosmids, yeast artificial chromosomes (YACS) and inter-species hybrid cells.

A base sequence at any point in the genome can be classified as either "single-copy" or "repetitive". For practical purposes the sequence needs to be long enough so that a complementary probe sequence can form a stable hybrid with the target sequence under the hybridization conditions being used. Such a length is typically in the range of several tens to hundreds of nucleotides.

A "single-copy sequence" is that wherein only one copy of the target nucleic acid sequence is present in the haploid genome. "Single-copy sequences" are also known in the art as "unique sequences". A "repetitive sequence" is that wherein there are more than one copy of the same target nucleic acid sequence in the genome. Each copy of a repetitive sequence need not be identical to all the others. The important feature is that the sequence be sufficiently similar to the other members of the family of repetitive sequences such that under the hybridization conditions being used, the same fragment of probe nucleic acid is capable of forming stable hybrids with each copy. A "shared repetitive sequence" is a sequence with some copies in the target region of the genome, and some elsewhere.

When the adjectives "single-copy", "repetitive", "shared repetitive", among other such modifiers, are used to describe sequences in the probe, they refer to the type of sequence in the target to which the probe sequence will bind. Thus, "a repetitive probe" is one that binds to a repetitive sequence in the target; and "a single-copy probe" binds to a single-copy target sequence.

Repetitive sequences occur in multiple copies in the haploid genome. The number of copies can range from two to hundreds of thousands, wherein the Alu family of repetitive DNA are exemplary of the latter numerous variety. The copies of a repeat may be clustered or interspersed throughout the genome. Repeats may be clustered in one or more locations in the genome, for example, repetitive sequences occurring near the centromeres of each chromosome, and variable number tandem repeats (VNTRs) [Nakamura et al, Science 235:1616 (1987)]; or the repeats may be distributed over a single chromosome [for example, repeats found only on the X chromosome as described by Bardoni et al., Cytogenet. Cell Genet., 46:575 (1987)]; or the repeats may be distributed over all the chromosomes, for example, the Alu family of repetitive sequences.

Herein, the terms repetitive sequences, repeated sequences and repeats are used interchangeably.

Shared repetitive sequences can be clustered or interspersed. Clustered repetitive sequences include tandem repeats which are so named because they are contiguous on the DNA molecule which forms the backbone of a chromosome. Clustered repeats are associated with well-defined regions of one or more chromosomes, e.g., the centromeric region. If one or more clustered repeats form a sizable fraction of a chromosome, and are shared with one or more non-target regions of the genome and are consequently removed from the heterogeneous mixture of fragments employed in the invention or the hybridization capacity thereof is disabled, perfect uniformity of staining of the target region may not be possible. That situation is comprehended by the use of the term "substantially uniform" in reference to the binding of the heterogeneous mixture of labeled nucleic acid fragments to the target.

Chromosome-specific staining of the current invention is accomplished by using nucleic acid fragments that hybridize to sequences specific to the target. These sequences may be either single-copy or repetitive, wherein the copies of the repeat occur predominantly in the target area. If nucleic acid fragments complementary to non-target regions of the genome are included in the probe, for example, shared repetitive sequences or non-specific sequences, their hybridization capacity needs to be sufficiently disabled or their prevalence sufficiently reduced, so that adequate staining contrast can be obtained.

The nucleic acid probes of this invention need not be absolutely specific for the targeted portion of the genome. They are intended to produce "staining contrast". "Contrast" is quantified by the ratio of the stain intensity of the target region of the genome to that of the other portions of the genome. For example, a DNA library produced by cloning a particular chromosome, such as those listed in Table I, can be used as a probe capable of staining the entire chromosome. The library contains sequences found only on that chromosome, and sequences shared with other chromosomes. In a simplified (approximately true to life) model of the human genome, about half of the chromosomal DNA falls into each class. If hybridization with the whole library were capable of saturating all of the binding sites, the target chromosome would be twice as bright (contrast ratio of 2) as the others since it would contain signal from the specific and shared sequences in the probe, whereas the other chromosome would only have signal from the shared sequences. Thus, only a modest decrease in hybridization of the shared sequences in the probe would substantially enhance the contrast. Contaminating sequences which only hybridize to non-targeted sequences, for example, impurities in a library, can be tolerated in the probe to the extent that said sequences do not reduce the staining contrast below useful levels.

In reality all of the target sites may not be saturated during the hybridization, and many other mechanisms contribute to producing staining contrast, but this model illustrates one general consideration in using probes targeted at a large portion of a genome.

The required contrast depends on the application for which the probe is designed. When visualizing chromosomes and nuclei, etc., microscopically, a contrast ratio of two or greater is often sufficient for identifying whole chromosomes. The smaller the individual segments of the target region, the greater the contrast needs to be to permit reliable recognition of the target relative to the fluctuations in staining of the non-targeted regions. When quantifying the amount of target region present in a cell nucleus by fluorescence intensity measurements using flow cytometry or quantitative microscopy, the required contrast ratio is on the order of $1/T$ or greater on average for the genome, where $T$ is the fraction of the genome contained in the targeted region. When the contrast ratio is equal to $1/T$, half of the total fluorescence intensity comes from the target region and half from the rest of the genome. For example, when using a high complexity probe for chromosome 1, which comprises about 10% of the genome, the required contrast ratio is on the order of 10, that is, for the chromosome 1 fluorescence intensity to equal that of the rest of the genome.

Background staining by the probe, that is, to the non-target region of the genome, may not be uniform. This degree of non-specificity may or may not inhibit the probe's use for a specific application. In some cases, removal of or further disabling the hybridization capacity of the probe fragments that bind to non-target sequences may be necessary.

For other applications, repetitive sequences that bind to centromeres, for example, alpha-satellite sequences, and/or telomeres can be part of the chromosome-specific staining reagents wherein the target includes some or all of the centromeres and/or telomeres in a genome along with perhaps other chromosomal regions. Exemplary of such an application would be that wherein the staining reagent is designed to detect random structural aberrations caused by clastogenic agents that result in dicentric chromosomes and other structural abnormalities, such as translocations. For example, addition of sequences which bind to all centromeres in a genome could allow more reliable distinguishing between dicentrics and translocations.

Application of staining reagents of this invention to a genome results in a substantially uniform distribution of probe hybridized to the targeted regions of a genome. The distribution of bound probe is deemed "substantially uniform" if the targeted regions of the genome can be visualized with useful contrast. For example, a target is substantially uniformly stained in the case wherein it is a series of visually separated loci if most of the loci are visible in most of the cells.

"Substantial portions" in reference to the base sequences of nucleic acid fragments that are complementary to chromosomal DNA means that the complementarity is extensive enough so that the fragments form stable hybrids with the chromosomal DNA under the hybridization conditions used. In particular, the term comprehends the situation where the nucleic acid fragments of the heterogeneous mixture possess some regions of sequence that are not perfectly complementary to target chromosomal material. The stringency can be adjusted to control the precision of the complementarity required for hybridization.

The phrase "metaphase chromosomes" is herein defined to mean not only chromosomes condensed in the metaphase stage of mitosis but includes any condensed chromosomes, for example, those condensed by premature chromosome condensation.

To disable the hybridization capacity of a nucleic acid sequence is herein sometimes abbreviated as "disabling the nucleic acid sequence".

The methods and reagents of this invention find a particularly appropriate application in the field of diagnostic cytogenetics, particularly in the field of diagnostic interphase cytogenetics. Detecting genetic rearrangements that are associated with a disease, such as cancer, are a specific application of the chromosome specific reagents and staining methods of this invention.

Contiguous gene syndromes are an example of the genetic rearrangements that the probes and methods of this invention can identify. Contiguous gene syndromes are characterized by the presence of several closely spaced genes which are in multiple and/or reduced copy number. Down syndrome is an example of a contiguous gene syndrome wherein an extra copy of a chromosomal region containing several genes is present.

Particularly described herein in Sections VI and VII are applications of chromosome-specific reagents and methods for detecting genetic rearrangements involving chromosomes 3, 13 and 17. Section VI concerns the retinoblastoma tumor suppressor gene (Rb-1), its mapping to 13q14 by use of chromosome-specific staining and digital image analysis, methods to detect deletions including those in subregions involving the Rb-1 locus (13q deletions) in both metaphase chromosomes and interphase nuclei, and the use of a reference probe, represented by a 13/21 pericentromeric alpha satellite probe within the chromosome-specific probe. The experiments were done with both cultured cells and clinical breast cancer samples.

Section VII concerns the detection of chromosome 3 and chromosome 17 aberrations associated with cancer. Centromeric-specific alpha satellite probes specific to chromosome 3 and chromosome 17 were used in conjunction with a 3p cosmid probe amplified and labeled by the PCR adapter-linker method, a 3q cosmid probe and a whole chromosome 3-specific probe. Experiments with these probes using a variety of labels showed chromosome aberrations, e.g., aneuploidy, deletions and/or translocations, in cancer cells in both metaphase spreads and interphase nuclei.

The following sections provide examples of making and using the staining compositions of this invention and are for purposes of illustration only and not meant to limit the invention in any way. The following abbreviations are used.

Abbreviations

| | |
|---|---|
| AAF | - N-acetoxy-N-2-acetyl-aminofluorene |
| BN | - bicarbonate buffer with NP-40 |
| BRL | - Bethesda Research Laboratories |
| bp | - base pair |
| CML | - chronic myelogenous leukemia |
| DAPI | - 4,6-diamidino-2-phenylindole |
| dATP | - deoxyadenosine triphosphate |
| DCS | - as in fluorescein-avidin DCS (a commercially available cell sorter grade of fluorescein Avidin D) |
| dCTP | - deoxycytosine triphosphate |
| dGPT | - deoxyguanosine triphosphate |
| DI | - DNA index |
| dNTP | - deoxynucleotide triphosphate |
| dTTP | - deoxythymidine triphosphate |
| DUTP | - deoxyridine triphosphate |
| EDTA | - ethylenediaminetetraacetate |
| FISH | - fluorescence in situ hybridization |
| FACS | - fluorescence-activated cell sorting |
| FITC | - fluorescein isothiocyanate |
| HPLC | - high performance liquid chromatography |
| IB | - isolation buffer |
| kb | - kilobase |
| kDa | - kilodalton |
| ml | - milliliter |
| mM | - milliMole |
| mm | - millimeter |
| ng | - nanogram |
| NP-40 | - non-ionic detergent commercially available from Sigma as Nonidet P-40 (St. Louis, MO) |
| PBS | - phosphate-buffered saline |
| PHA | - phytohemagglutinin |
| PCR | - polymerase chain reaction |
| PI | - propidium iodide |
| PMSF | - phenylmethylsulfonyl fluoride |
| PN buffer | - mixture of 0.1 M $NaH_2PO_4$ and 0.1 M $Na_2HPO_4$, pH 8; 0.1% NP-40 |
| PNM buffer | - Pn buffer plus 5% nonfat dry milk (centrifuged); 0.02% Na azide |
| Rb-1 | - retinoblastoma tumor suppressor gene |

| RFLP | - restriction fragment length polymorphism |
| SD | - Standard Deviation |
| SDS | - sodium dodecyl sulfate |
| SSC | - 0.15 M NaCl/0.015 M Na citrate, pH 7 |
| ug | - microgram |
| ul | - microliter |
| um | - micrometer |
| uM | - micromole |
| VNTR | - variable number tandem repeat |

## I. Methods of Preparing Chromosome-Specific Staining Reagents

### I.A. Isolation of Chromosome-Specific DNA and Formation of DNA Fragment Libraries.

The first step in a preferred method of making the compositions of the invention is isolating chromosome-specific DNA (which term includes target-specific and/or region-specific DNA, as indicated above, wherein specific refers to the origin of the DNA). This step includes first isolating a sufficient quantity of the particular chromosome type or chromosomal subregion to which the staining composition is directed, then extracting the DNA from the isolated chromosome(s) or chromosomal subregion(s). Here "sufficient quantity" means sufficient for carrying out subsequent steps of the method. Preferably, the extracted DNA is used to create a library of DNA inserts by cloning using standard genetic engineering techniques.

Preferred cloning vectors include, but are not limited to, yeast artificial chromosomes (YACS), plasmids, bacteriophages and cosmids. Preferred plasmids are Bluescribe plasmids; preferred bacteriophages are lambda insertion vectors, more preferably Charon 4A, Charon 21A, Charon 35, Charon 40 and GEM11; and preferred cosmids include Lawrist 4, Lawrist 5 and sCos1.

As indicated above, the DNA can be isolated from any source. Chromosome-specific staining reagents can be made from both plant and animal DNA according to the methods of this invention. Important sources of animal DNA are mammals, particularly primates or rodents wherein primate sources are more particularly human and monkey, and rodent sources are more particularly rats or mice, and more particularly mice.

### 1. Isolating DNA from an Entire Chromosome.

A preferred means for isolating particular whole chromosomes (specific chromosome types) is by direct flow sorting [fluorescence-activated cell sorting (FACS)] of metaphase chromosomes with or without the use of interspecific hybrid cell systems. For some species, every chromosome can be isolated by currently available sorting techniques. Most, but not all, human chromosomes are currently isolatable by flow sorting from human cells, Carrano et al., "Measurement and Purification of Human Chromosomes by Flow Cytometry and Sorting," PNAS, 76: 1382 (1979). Thus, for isolation of some human chromosomes, use of the human/rodent hybrid cell system may be necessary, see Kao, "Somatic Cell Genetics and Gene Mapping," International Review of Cytology, 85: 109-146 (1983), for a review, and Gusella et al., "Isolation and Localization of DNA Segments from Specific Human Chromosomes," PNAS, 77: 2829-2833 (1980). Chromosome sorting can be done by commercially available fluorescence-activated sorting machines, e.g., Becton Dickinson FACS-II, Coulter Epics V sorter, or special purpose sorters optimized for chromosome sorting or like instrument.

DNA is extracted from the isolated chromosomes by standard techniques, e.g., Marmur, "A Procedure for the Isolation of Deoxyribonucleic Acid from Micro-Organisms," J. Mol. Biol., 3: 208-218 (1961); or Maniatis et al., Molecular cloning: A Laboratory Manual (Cold Spring Harbor Laboratory, 1982) pgs. 280-281. These references are incorporated by reference for their descriptions of DNA isolation techniques.

Generation of insert libraries from the isolated chromosome-specific DNA is carried out using standard genetic engineering techniques, e.g., Davies et al., "Cloning of a Representative Genomic Library of the Human X Chromosome After Sorting by Flow Cytometry," Nature, 293: 374-376 (1981); Krumlauf et al., "Construction and Characterization of Genomic Libraries from Specific Human Chromosomes," PNAS, 79: 2971-2975 (1982); Lawn et al., "The Isolation and Characterization of Linked Delta-and-Beta-Globin Genes from a Cloned Library of Human DNA." Cell, 15: 1157-1174 (1978); and Maniatis et al., "Molecular cloning: A Laboratory Manual," (Cold Springs Harbor Laboratory, 1982), pgs. 256-308; Van Dilla et al., Biotechnology, 4: 537 (1986); Fuscoe, Gene, 52:291 (1987); and Fuscoe et al., Cytogenet. Cell Genet., 43:79 (1986).

Recombinant DNA libraries for each of the human chromosomes have been constructed by the National Laboratory Gene Library Project and are available from the American Type Culture Collection. [Van Dilla et al., Biotechnology, 4:537 (1986).] Small insert-containing libraries were constructed by complete digestion of flow sorted human chromosome genomic DNA with HindIII or EcoRI and cloning into the Lambda insertion vector Charon 21A. The vector is capable of accepting human inserts of up to 9.1 kb in size. Thus, HindIII (or EcoRI) restriction fragments greater than 9.1 kb will not be recovered from these libraries. The observed average insert size in these libraries is approximately 4 kb. A representative list of the HindIII chromosome-specific libraries with their ATCC accession numbers are shown in Table 1.

### TABLE 1
### HUMAN CHROMOSOME - SPECIFIC GENOMIC LIBRARIES
### IN CHARON 21A VECTOR

| CHROMOSOME | ATCC # | LIBRARY |
|---|---|---|
| 1 | 57753 | LL01NS01 |
| 1 | 57754 | LL01NS02 |
| 2 | 57744 | LL02NS01 |
| 3 | 57751 | LL03NS01 |
| 4 | 57700 | LL04NS01 |
| 4 | 57745 | LL04NS02 |
| 5 | 57746 | LL05NS01 |
| 6 | 57701 | LL06NS01 |
| 7 | 57755 | LL07NS01 |
| 8 | 57702 | LL08NS02 |
| 9 | 57703 | LL09NS01 |
| 10 | 57736 | LL10NS01 |
| 11 | 57704 | LL11NS01 |
| 12 | 57756 | LL12NS01 |
| 13 | 57705 | LL13NS01 |
| 13 | 57757 | LL13NS02 |
| 14 | 57706 | LL14NS01 |
| 14/15 | 57707 | LL99NS01 |
| 15 | 57737 | LL15NS01 |
| 16 | 57758 | LL16NS03 |
| 17 | 57759 | LL17NS02 |
| 18 | 57710 | LL18NS01 |
| 19 | 57711 | LL19NS01 |
| 20 | 57712 | LL20NS01 |
| 21 | 57713 | LL21NS02 |
| 22 | 57714 | LL22NS01 |
| X | 57747 | LL0XNS01 |
| Y | 57715 | LL0YNS01 |

Alternatively, the extracted DNA from a sorted chromosome type can be amplified by the polymerase chain reaction (PCR) rather than cloning the extracted DNA in a vector or propagating it in a cell line. Appropriate tails are added to the extracted DNA in preparation for PCR. References for such PCR procedures are set out in Section I.B infra.

Other possible methods of isolating the desired sequences from hybrid cells include those of Schmeckpeper et al., "Partial Purification and Characterization of DNA from Human X Chromosome," PNAS, 76: 6525-6528 (1979); and Olsen et al., Biochem., 19: 2419-2428 (1980).

## 2. Isolating DNA from a Portion of a Chromosome.

Among the methods that can be used for isolating region-specific chromosomal DNA include the selection of an appropriate chromosomal region from DNA that has previously been mapped, for example, from a library of mapped cosmids; the sorting of derivative chromosomes, for example, by FACS; the microdissection of selected chromosomal material; subtractive hybridization; identification of an appropriate hybrid cell containing a desired chromosomal fragment, extracting and amplifying the DNA, and selecting the desired amplified DNA; and the selection of appropriate chromosomal material from radiation hybrids. The standard genetic engineering techniques outlined above in subsection I.A.1 are used in such procedures well-known to those in the art. Amplification of the region-specific DNA can be performed by cloning in an appropriate vector, propagating in an appropriate cell line, and/or by the use of PCR (see I.B infra).

A preferred method of isolating chromosomal region-specific DNA is to use mapped short DNA sequences to probe a library of longer DNA sequences, wherein the latter library has usually been cloned in a different vector. For example, a probe cloned in a plasmid can be used to probe a cosmid or yeast artificial chromosome (YAC) library. By using an initial seed probe, overlapping clones in the larger insert library can be found (a process called "walking"), and a higher complexity probe can be produced for reliable staining of the chromosomal region surrounding the seed probe. Ultimately, when an entire genome for a species has been mapped (for example, by the Human Genome Project for the human species), ordered clones for the entire genome of the species will be available. One can then easily select the appropriate clones to form a probe of the desired specificity.

Another method of isolating DNA from a chromosomal region or regions (or also a whole chromosome) is to propagate such a chromosomal region or regions in an appropriate cell line (for example, a hybrid cell line such as a human/hamster hybrid cell), extract the DNA from the cell line and clone it in an appropriate vector and select clones containing human DNA to form a library. When a hybrid cell is used, the chromosomes in the hybrid cell containing the human chromosomal material may be separated by flow sorting (FACS) prior to cloning to increase the frequency of human clones in the library. Still further, total DNA from the hybrid cell can be isolated and labeled without further cloning and used as a probe.

## 3. Single-Stranded Probes.

In some cases, it is preferable that the nucleic acid fragments of the heterogeneous mixture consist of single-stranded RNA or DNA. Under some conditions, the binding efficiency of single-stranded nucleic acid probes has been found to be higher during in situ hybridization, e.g., Cox et al., "Detection of mRNAs in Sea Urchin Embryos by In Situ Hybridization Using Asymmetric RNA Probes," Develop. Biol., 101: 485-502 (1984).

Standard methods are used to generate RNA fragments from isolated DNA fragments. For example, a method developed by Green et al., described in Cell, 32: 681-694 (1983), is commercialy available from Promega Biotec [Madison, WI(USA)] under the tradename "Riboprobe." Other transcription kits suitable for use with the present invention are available from United States Biochemical Corporation [Cleveland, OH (USA)] under the tradename "Genescribe." Single-stranded DNA probes can be produced with the single-stranded bacteriophage M13, also available in kit form, e.g. Bethesda Research Laboratories [Gaithersburg, MD (USA)]. Hybridizations have been performed with the libraries of Table 1 subcloned into the Bluescribe plasmid vector [Stratagene, La Jolla, CA (USA)] as described in Pinkel et al., PNAS (USA), 85: 9138 (1988). The Bluescribe plasmid contains RNA promoters which permit production of single-stranded probes.

U.S. Patent No. 5,028,525 (issued July 2, 1991) entitled "Method of Preparing and Applying Single Stranded DNA Probes to Double Stranded Target DNAs In Situ," provides methods for preparing and applying non-self-complementary single-stranded nucleic acid probes that improve signal-to-noise ratios attainable in in situ hybridization by reducing non-specific and mismatched binding of the probe. That patent further provides for methods of denaturing double-stranded target nucleic acid which minimizes single-stranded regions available for hybridization that are non-complementary to probe sequences. Briefly, probe is constructed by treating DNA with a restriction enzyme and an exonuclease to form template/primers for a DNA polymerase. The digested strand is resynthesized in the presence of labeled nucleoside triphosphate precursor, and the labeled single-stranded fragments are separated from the resynthesized fragments to form the probe. The target nucleic acid is treated with the same restriction enzyme used to construct the probe, and is treated with an exonuclease before application of the probe.

I.B. PCR

Another method of producing probes of this invention includes the use of the polymerase chain reaction [PCR]. [For an explanation of the mechanics of PCR, see Saiki et al., Science, 230: 1350 (1985) and U.S. Patent Nos. 4,683,195, 4,683,202 (both issued July 28, 1987) and 4,800,159 (issued January 24, 1989).] PCR offers a rapid, sensitive and versatile cell-free molecular cloning system in which only minute amounts of starting material are required. As shown infra, PCR adapter-linker amplification [Saunders et al., Nuc. Acids Res., 17: 9027; Johnson, Genomics, 6: 243 (1990)] can be used to produce chromosome-specific painting probes of this invention. [See PCT 90/00434 (published August 9, 1990).]

Target-specific nucleic acid sequences, isolated as indicated above, can be amplified by PCR to produce target-specific sequences which are reduced in or free of repetitive sequences. The PCR primers used for such a procedure are for the ends of the repetitive sequences, resulting in amplification of sequences flanked by the repeats.

One can further suppress production of repetitive sequences in such a PCR procedure by first hybridizing complementary sequences to said repetitive sequence wherein said complementary sequences have extended non-complementary flanking ends or are terminated in nucleotides which do not permit extension by the polymerase. The non-complementary ends of the blocking sequences prevent the blocking sequences from acting as a PCR primer during the PCR process. Primers directed against the Alu and L1 repetitive DNA families have allowed the selective amplification of human sequences by interspersed repetitive sequence PCR (IRS-PCR) [Nelson et al., PNAS, 86: 6686 (1989); Ledbetter et al., Genomics, 6: 475 (1990)].

II. Removal of Repetitive Sequences and/or Disabling the Hybridization Capacity of Repetitive Sequences

Typically a probe of the current invention is produced in a number of steps including: obtaining source nucleic acid sequences that are complementary to the target region of the genome, labeling and otherwise processing them so that they will hybridize efficiently to the target and can be detected after they bind, and treating them to either disable the hybridization capacity or remove a sufficient proportion of shared repetitive sequences, or both disable and remove such sequences. The order of these steps depends on the specific procedures employed.

The following methods can be used to remove shared repetitive sequences and/or disable the hybridization capacity of such shared repetitive sequences. Such methods are representative and are expressed schematically in terms of procedures well known to those of ordinary skill the art, and which can be modified and extended according to parameters and procedures well known to those in the art.

1. Single-copy probes.

A single-copy probe consists of nucleic acid fragments that are complementary to single-copy sequences contained in the target region of the genome. One method of constructing such a probe is to start with a DNA library produced by cloning the target region. Some of the clones in the library will contain DNA whose entire sequence is single-copy; others will contain repetitive sequences; and still others will have portions of single-copy and repetitive sequences. Selection, on a clone by clone basis, and pooling of those clones containing only single-copy sequences will result in a probe that will hybridize specifically to the target region. The single-copy nature of a clone can ultimately be established by Southern hybridization using standard techniques.

Southern analysis is very time consuming and labor intensive. Therefore, less perfect but more efficient screening methods for obtaining candidate single-copy clones are useful. Fuscoe et al., Genomics, 5: 100-109 (1989) provides efficient procedures for selecting large numbers of single-copy sequence or very low copy number repeat sequence clones.

A disadvantage of clone by clone procedures is that a clone is discarded even if only a portion of the sequence it contains is repetitive. The larger the length of the cloned nucleic acid, the greater the chance that it will contain a repetitive sequence. Therefore, when nucleic acid is propagated in a vector that contains large inserts such as a cosmid, YAC, or in a cell line, such as hybrid cells, it may be advantageous to subclone it in smaller pieces before the single-copy selection is performed. The selection procedures just outlined above do not discriminate between shared and specific repetitive sequences; clones with detectable repetitive sequences of either type are not used in the probe.

## 2. Individual testing of hybridization properties.

The hybridization specificity of a piece of nucleic acid, for example, a clone, can be tested by in situ hybridization. If under appropriate hybridization conditions it binds to single-copy or repetitive sequences specific for the desired target region, it can be included in the probe. Many sequences with specific hybridization characteristics are already known, such as chromosome-specific repetitive sequences [Trask et al., Hum. Genet., 78: 251 (1988) and references therein], VNTRs, numerous mapped single copy sequences. More are continuously being mapped. Such sequences can be included in a probe of this invention.

## 3. Bulk Procedures.

In many genomes, such as the human genome, a major portion of shared repetitive DNA is contained in a few families of highly repeated sequences such as Alu. A probe that is substantially free of such high-copy repetitive sequences will produce useful staining contrast in many applications. Such a probe can be produced from some source of nucleic acid sequences, for example, the libraries of Table I, with relatively simple bulk procedures. Therefore, such bulk procedures are the preferred methods for such applications.

These methods primarily exploit the fact that the hybridization rate of complementary nucleic acid strands increases as their concentration increases. Thus, if a heterogeneous mixture of nucleic acid fragments is denatured and incubated under conditions that permit hybridization, the sequences present at high concentration will become double-stranded more rapidly than the others. The double-stranded nucleic acid can then be removed and the remainder used as a probe. Alternatively, the partially hybridized mixture can be used as the probe, the double-stranded sequences being unable to bind to the target. The following are methods representative of bulk procedures that are useful for producing the target-specific staining of this invention.

## 3a. Self-reassociation of the probe.

Double-stranded probe nucleic acid in the hybridization mixture is denatured and then incubated under hybridization conditions for a time sufficient for the high-copy sequences in the probe to become substantially double-stranded. The hybridization mixture is then applied to the sample. The remaining labeled single-stranded copies of the highly repeated sequences bind throughout the sample producing a weak, widely distributed signal. The binding of the multiplicity of low-copy sequences specific for the target region of the genome produce an easily distinguishable specific signal.

## 3b. Use of blocking nucleic acid.

Unlabeled nucleic acid sequences which are complementary to those sequences in the probe whose hybridization capacity it is desired to inhibit are added to the hybridization mixture. The probe and blocking nucleic acid are denatured, if necessary, and incubated under appropriate hybridization conditions. The sequences to be blocked become double-stranded more rapidly than the others, and therefore are unable to bind to the target when the hybridization mixture is applied to the target. In some cases, the blocking reaction occurs so quickly that the incubation period can be very short, and adequate results can be obtained if the hybridization mix is applied to the target immediately after denaturation. A blocking method is generally described by Sealy et al., "Removal of Repeat Sequences form Hybridization Probes", Nucleic Acid Research, 13:1905 (1985). Examples of blocking nucleic acids include genomic DNA, a high-copy fraction of genomic DNA and particular sequences as outlined below (i-iii).

## 3b.i. Genomic DNA.

Genomic DNA contains all of the nucleic acid sequences of the organism in proportion to their copy-number in the genome. Thus, adding genomic DNA to the hybridization mixture increases the concentration of the high-copy repeat sequences more than low-copy sequences, and therefore is more effective at blocking the former. However, the genomic DNA does contain copies of the sequences that are specific to the target and so will also reduce the desired chromosome-specific binding if too much is added. Guidelines to determine how much genomic DNA to add and timing of the additions are discussed in EP Pub. No. 430,402 (published June 5, 1991).

## 3b.ii. High-copy fraction of genomic DNA.

The difficulty with use of genomic DNA is that it also blocks the hybridization of the low-copy sequences, which are predominantly the sequences that give the desired target staining. Thus, fractionating the genomic DNA to obtain only the high-copy sequences and using them for blocking overcomes this difficulty. Such fractionation can be done, for example, with hydroxyapatite as described below (3c.i).

3b.iii. Specified sequences.

The blocking of a particular sequence in the probe can be accomplished by adding many unlabeled copies of that sequence. For example, Alu sequences in the probe can be blocked by adding cloned Alu DNA. Blocking DNA made from a mixture of a few clones containing the highest copy sequences in the human genome can be used effectively with chromosome-specific libraries for example, those of Table I. Alternatively, unlabeled nucleic acid sequences from one or more chromosome-pecific libraries could be used to block a probe containing labeled sequences from one or more other chromosome-specific libraries. The shared sequences would be blocked whereas sequences occurring only on the target chromosome would be unaffected.

3c. Removal of Sequences.

3c.i. Hydroxyapatite.

Single- and double-stranded nucleic acids have different binding characteristics to hydroxyapatite. Such characteristics provide a basis commonly used for fractionating nucleic acids. Hydroxyapatite is commerically available [e.g., Bio-Rad Laboratories, Richmond, CA (USA)]. The fraction of genomic DNA containing sequences with a particular degree of repetition, from the highest copy-number to single-copy, can be obtained by denaturing genomic DNA, allowing it to reassociate under appropriate conditions to a particular value of $C_0t$, followed by separation using hydroxyapatite. The single- and double-stranded nucleic acid can also be discriminated by use of S1 nuclease. Such techniques and the concept of $C_0t$ are explained in Britten et al., "Analysis of Repeating DNA Sequences by Reassociation", in Methods in Enzymology, 29: 363-418 (1974).

The single-stranded nucleic acid fraction produced in 3a. or 3b. above can be separated by hydroxyapatite and used as a probe. Thus, the sequences that have been blocked (that become double-stranded) are physically removed. The probe can then be stored until needed. The probe can then be used without additional blocking nucleic acid, or its staining contrast can perhaps be improved by additonal blocking.

3c.ii. Reaction with immobilized nucleic acid.

Removal of particular sequences can also be accomplished by attaching single-stranded "absorbing" nucleic acid sequences to a solid support. Single-stranded source nucleic acid is hybridized to the immobilized nucleic acid. After the hybridization, the unbound sequences are collected and used as the probe. For example, human genomic DNA can be used to absorb repetitive sequences from human probes. One such method is described by Brison et al., "General Method for Cloning Amplified DNA by Differential Screening with Genomic Probes," Molecular and Cellular Biology, 2: 578-587 (1982). Briefly, minimally sheared human genomic DNA is bound to diazonium cellulose or a like support. The source DNA, appropriately cut into fragments, is hybridized against the immobilized DNA to $C_0t$ values in the range of about 1 to 100. The preferred stringency of the hybridization conditions may vary depending on the base composition of the DNA. Such a procedure could remove repetitive sequences from chromosome-specific libraries, for example, those of Table I, to produce a probe capable of staining a whole human chromosome.

3d. Blocking non-targeted sequences in the targeted genone.

Blocking of non-targeted binding sites in the targeted genome by hybridization with unlabeled complementary sequences will prevent binding of labeled sequences in the probe that have the potential to bind to those sites. For example, hybridization with unlabeled genomic DNA will render the high-copy repetitive sequences in the target genome double-stranded. Labeled copies of such sequences in the probe will not be able to bind when the probe is subsequently applied.

In practice, several mechanisms combine to produce the staining contrast. For example, when blocking DNA is added to the probe as in 3b above, that which remains single-stranded when the probe is applied to the target can bind to and block the target sequences. If the incubation of the probe with the blocking DNA is minimal, then the genomic DNA simultaneously blocks the probe and competes with the probe for binding sites in the target.

III. Labeling the Nucleic Acid Fragments of the Heterogeneous Mixture.

Several techniques are available for labeling single- and double-stranded nucleic acid fragments of the heterogeneous mixture. They include incorporation of radioactive labels, e.g. Harper et al. Chromosoma, 83: 431-439 (1984); direct attachment of fluorochromes or enzymes, e.g. Smith et al., Nuc. Acids Res., 13: 2399-2412 (1985), and Connolly et al., Nuc. Acids Res., 13: 4485-4502 (1985); and various chemical modifications of the nucleic acid fragments that render them detectable immunochemically or by other affinity reactions, e.g. Tchen et al., "Chemically Modified Nucleic Acids as Immunodetectable Probes in Hybridization Experiments," PNAS, 81: 3466-3470 (1984); Richardson et al., "Biotin and Fluorescent Labeling of RNA Using T4 RNA Ligase," Nuc. Acids Res., 11: 6167-6184 (1983); Langer et al., "Enzymatic Synthesis of Biotin-Labeled Polynucleotides: Novel Nucleic Acid Affinity Probes," PNAS, 78: 6633-6637 (1981); Brigati et al., "Detection of Viral Genomes in Cultured Cells and Paraffin-Embedded Tissue Sections Using Biotin-Labeled Hybridization Probes," Virol., 126: 32-50 (1983); Broker et al., "Electron Microscopic Visualization of tRNA Genes with Ferritin-Avidin: Biotin Labels," Nuc. Acids Res., 5: 363-384 (1978); Bayer et al., "The Use of the Avidin Biotin Complex as a Tool in Molecular Biology," Methods of Biochem. Analysis, 26: 1-45 (1980); Kuhlmann, Immunoenzyme Techniques in Cytochemistry (Weinheim, Basel, 1984). Langer-Safer et al., PNAS (USA), 79: 4381 (1982): Landegent et al., Exp. Cell Res., 153: 61 (1984); and Hopman et al., Exp. Cell Res., 169: 357 (1987).

Exemplary labeling means include those wherein the probe fragments are biotinylated, modified with N-acetoxy-N-2-acetylaminofluorene, modified with fluorescein isothiocyanate, modified with mercury/TNP ligand, sulfonated, digoxigenenated or contain T-T dimers.

The key feature of "probe labeling" is that the probe bound to the target be detectable. In some cases, an intrinsic feature of the probe nucleic acid, rather than an added feature, can be exploited for this purpose. For example, antibodies that specifically recognize RNA/DNA duplexes have been demonstrated to have the ability to recognize probes made from RNA that are bound to DNA targets [Rudkin and Stollar, Nature, 265:472-473 (1977)]. The RNA used for such probes is unmodified. Probe nucleic acid fragments can be extended by adding "tails" of modified nucleotides or particular normal nucleotides. When a normal nucleotide tail is used, a second hybridization with nucleic acid complementary to the tail and containing fluorochromes, enzymes, radioactivity, modified bases, among other labeling means, allows detection of the bound probe. Such a system is commerically available from Enzo Biochem [Biobridge Labeling System; Enzo Biochem Inc., New York, N.Y.(USA)].

Another example of a means to visualize the bound probe wherein the nucleic acid sequences in the probe do not directly carry some modified constituent is the use of antibodies to thymidine dimers. Nakane et al., ACTA Histochem. Cytochem., 20 (2):229 (1987), illustrate such a method wherein thymine-thymine dimerized DNA (T-T DNA) was used as a marker for in situ hybridization. The hybridized T-T DNA was detected immunohistochemically using rabbit anti-T-T DNA antibody.

All of the labeling techniques disclosed in the above references may be preferred under particular circumstances. Further, any labeling techniques known to those in the art would be useful to label the staining compositions of this invention. Several factors govern the choice of labeling means, including the effect of the label on the rate of hybridization and binding of the nucleic acid fragments to the chromosomal DNA, the accessibility of the bound probe to labeling moieties applied after initial hybridization, the mutual compatibility of the labeling moieties, the nature and intensity of the signal generated by the label, the expense and ease in which the label is applied, and the like.

Several different high complexity probes, each labeled by a different method, can be used simultaneously. The binding of different probes can thereby be distinguished, for example, by different colors.

IV. In Situ Hybridization.

Application of the heterogeneous mixture of the invention to chromosomes is accomplished by standard in situ hybridization techniques. Several excellent guides to the technique are available, e.g., Gall and Pardue, "Nucleic Acid Hybridization in Cytological Preparations," Methods in Enzymology, 21: 470-480 (1981); Henderson, "Cytological Hybridization to Mammalian Chromosomes," International Review of Cytology, 76: 1-46 (1982); and Angerer et al., "In Situ Hybridization to Cellular RNAs," in Genetic Engineering: Principles and Methods, Setlow and Hollaender, Eds., Vol. 7, pgs. 43-65 (Plenum Press, New York, 1985).

Three factors influence the staining sensitivity of the hybridization probes: (1) efficiency of hybridization (fraction of target DNA that can be hybridized by probe), (2) detection efficiency (i.e., the amount of visible signal that can be obtained from a given amount of hybridization probe), and (3) level of noise produced by nonspecific binding of probe or components of the detection system.

Generally in situ hybridization comprises the following major steps: (1) fixation of tissue or biological structure to be examined, (2) prehybridization treatment of the biological structure to increase accessibility of target DNA, and to reduce nonspecific binding, (3) hybridization of the heterogeneous mixture of probe to the DNA in the biological structure or tissue; (4) posthybridization washes to remove probe not bound in specific hybrids, and (5) detection of the hybridized probes of the heterogeneous mixture. The reagents used in each of these steps and their conditions of use vary depending on the particular situation.

The following comments are meant to serve as a guide for applying the general steps listed above. Some experimentation may be required to establish optimal staining conditions for particular applications.

In preparation for the hybridization, the probe, regardless of the method of its production, may be broken into fragments of the size appropriate to obtain the best intensity and specificity of hybridization. As a general guideline concerning the size of the fragments, one needs to recognize that if the fragments are too long they are not able to penetrate into the target for binding and instead form aggregates that contribute background noise to the hybridization; however, if the fragments are too short, the signal intensity is reduced.

Under the conditions of hybridization wherein human genomic DNA is used, the preferred size range of the probe fragments is from about 200 bases to about 1000 bases, more preferably about 400 to 800 bases for double-stranded, nick-translated probes and about 200 to 600 bases for single-stranded or PCR adapter-linker probes. The preferred hybridization temperature is about 30°C to about 45°C, more preferably about 35°C to 40°C; the preferred washing temperature range is from about 40°C to about 50°C.

The size of the probe fragments is checked before hybridization to the target; preferably the size of the fragments is monitored by electrophoresis, more preferably by denaturing agarose gel electrophoresis.

Fixatives include acid alcohol solutions, acid acetone solutions, Petrunkewitsch's reagent, and various aldehydes such as formaldehyde, paraformaldehyde, glutaraldehyde, or the like. Preferably, ethanol-acetic acid or methanol-acetic acid solutions in about 3:1 proportions are used to fix the chromosomes in metaphase spreads. For cells or chromosomes in suspension, a fixation procedure disclosed by Trask, et al., in Science, 230: 1401-1402 (1985), is useful. Briefly, $K_2CO_3$ and dimethylsuberimidate (DMS) are added (from a 5x concentrated stock solution, mixed immediately before use) to a suspension containing about $5 \times 10^6$ nuclei/ml. Final $K_2CO_3$ and DMS concentrations are 20 mM and 3 mM, respectively. After 15 minutes at 25°C, the pH is adjusted from 10.0 to 8.0 by the addition of 50 microliters of 100 microliters of 100 mM citric acid per milliliter of suspension. Nuclei are washed once by centrifugation (300g, 10 minutes, 4°C in 50 mM kCl, 5 mM Hepes buffer, at pH 9.0, and 10 mM $MgS0_4$).

A preferred fixation procedure for cells or nuclei in suspension is disclosed by Trask et al., Hum. Genet., 78:251-259 (1988). Briefly, nuclei are fixed for about 10 minutes at room temperature in 1% paraformaldehyde in PBS, 50 mM $MgS0_4$, pH 7.6 and washed twice. Nuclei are resuspended in isolation buffer (IB) (50 mM KC1, 5 mM HEPES, 10 mM $MgSO_4$, 3 mM dithioerythritol, 0.15 mg/ml RNase, pH 8.0)/0.05% Triton X-100 at $10^8$/ml.

Frequently before in situ hybridization chromosomes are treated with agents to remove proteins. Such agents include enzymes or mild acids. Pronase, pepsin or proteinase K are frequently used enzymes. A represent-ative acid treatment is 0.02-0.2 N HCl, followed by high temperature (e.g., 70-C) washes. Optimization of deproteinization requires a combination of protease concentration and digestion time that maximizes hybridization, but does not cause unacceptable loss of morphological detail. Optimum conditions vary according to tissue types and method of fixation. Additional fixation after protease treatment may be useful. Thus, for particular applications, some experimentation may be required to optimize protease treatment.

In some cases pretreatment with RNase may be desirable to remove residual RNA from the target. Such removal can be accomplished by incubation of the fixed chromosomes in 50-100 microgram/milliliter RNase in 2X SSC (where SSC is a solution of 0.15M NaCL and 0.015M sodium citrate) for a period of 1-2 hours at room temperature.

The step of hybridizing the probes of the heterogeneous probe mixture to the chromosomal DNA involves (1) denaturing the target DNA so that probes can gain access to complementary single-stranded regions, and (2) applying the heterogeneous mixture under conditions which allow the probes to anneal to complementary sites in the target. Methods for denaturation include incubation in the presence of high pH, low pH, high temperature, or organic solvents such as formamide, tetraalkylammonium halides, or the like, at various combinations of concentration and temperature. Single-stranded DNA in the target can also be produced with enzymes, such as, Exonuclease III [van Dekken et al., Chromosoma (Berl) 97:1-5 (1988)]. The preferred denaturing procedure is incubation for between about 1-10 minutes in formamide at a concentration between about 35-95 percent in 2X SSC and at a temperature between about 25-70-C. Determination of the optimal incubation time, concentration, and temperature within these ranges depends on several variables, including the method of fixation and type of probe nucleic acid (for example, DNA or RNA).

After the chromosomal DNA is denatured, the denaturing agents are typically removed before application of the heterogeneous probe mixture. Where formamide and heat are the primary denaturing agents, removal is conveniently accomplished by several washes with a solvent, which solvent is frequently chilled, such as a 70%, 85%, 100% cold ethanol series. Alternatively the composition of the denaturant can be adjusted as appropriate for the in situ hybridization by addition of other consitutents or washes in appropriate solutions. The probe and target nucleic acid may be denatured simultaneously by applying the hybridization mixture and then heating to the appropriate temperature.

The ambient physiochemical conditions of the chromosomal DNA and probe during the time the heterogeneous mixture is applied is referred to herein as the hybridization conditions, or annealing conditions. Optimal hybridization conditions for particular applications can be adjusted by controlling several factors, including concentration of the constituents, incubation time of chromosomes in the heterogeneous mixture, and the concentrations, complexities, and lengths of the nucleic acid fragments making up the heterogeneous mixture. Roughly, the hybridization conditions must be sufficiently close to the melting temperature to minimize nonspecific binding. On the other hand, the conditions cannot be so stringent as to reduce correct hybridizations of complementary sequences below detectable levels or to require excessively long incubation times.

The concentrations of nucleic acid in the hybridization mixture is an important variable. The concentrations must be high enough so that sufficient hybridization of respective chromosomal binding sites occurs in a reasonable time (e.g., within hours to several days). Higher concentrations than that necessary to achieve adequate signals should be avoided so that nonspecific binding is minimized. An important practical constraint on the concentration of nucleic acid in the probe in the heterogeneous mixture is solubility. Upper bounds exist with respect to the fragment concentration, i.e., unit length of nucleic acid per unit volume, that can be maintained in solution and hybridize effectively.

The fixed target object can be treated in several ways either during or after the hybridization step to reduce nonspecific binding of probe DNA. Such treatments include adding nonprobe, or "carrier", DNA to the heterogeneous mixture, using coating solutions, such as Denhardt's solution (Biochem. Biophys. Res. Commun., 23: 641-645 (1966), with the heterogeneous mixture, incubating for several minutes, e.g., 5-20, in denaturing solvents at a temperature 5-10°C above the hybridization temperature, and in the case of RNA probes, mild treatment with single strand RNase (e.g., 5-10 micrograms per millileter RNase) in 2X SSC at room temperature for 1 hour).

### V. Specific Applications.

The present invention allows microscopic and in some cases flow cytometric detection of genetic abnormalities on a cell by cell basis. The microscopy can be performed entirely by human observers, or include various degrees of addititional instrumentation and computational assistance, up to full automation. The use of instrumentation and automation for such analyses offers many advantages. Among them are the use of fluorescent dyes that are invisible to human observers (for example, infared dyes), and the opportunity to interpret results obtained with multiple labeling methods which might not be simultaneously visible (for example, combinations of fluorescent and absorbing stains, autoradiography, etc.) Quantitative measurements can be used to detect differences in staining that are not detectable by human observers. As is described below, automated analysis can also increase the speed with which cells and chromosomes can be analysed.

The types of cytogenetic abnormalities that can be detected with the probes of this invention include: Duplication of all or part of a chromosome type can be detected as an increase in the number or size of distinct hybridization domains in metaphase spreads or interphase nuclei following hybridization with a probe for that chromosome type or region, or by an increase in the amount of bound probe. If the probe is detected by fluorescence, the amount of bound probe can be determined either flow cytometrically or by quantitative fluorescence microscopy. Deletion of a whole chromosome or chromosome region can be detected as a decrease in the number or size of distinct hybridization domains in metaphase spreads or interphase nuclei following hybridization with a probe for that chromosome type or region, or by a decrease in the amount of bound probe. If the probe is detected by fluorescence, the amount bound can be determined either flow cytometrically or by quantitative fluorescence microscopy. Translocations, dicentrics and inversions can be detected in metaphase spreads and interphase nuclei by the abnormal juxtaposition of hybridization domains that are normally separate following hybridization with probes that flank or span the region(s) of the chromosome(s) that are at the point(s) of rearrangement. Translocations involve at least two different chromosome types and result in derivative chromosomes possessing only one centromere each. Dicentrics involve at least two different chromosome types and result in at least one chromosome fragment lacking a centromere and one having two centromeres. Inversions involve a reversal of polarity of a portion of a chromosome.

### Tumor Cytogenetics

Numerous studies in recent years have revealed the existence of structural and numerical chromosome aberrations that are diagnostic for particular disease phenotypes and that provide clues to the genetic nature of the disease itself. Prominent examples include the close association between chronic myelogeneous leukemia and a translocation involving chromosome 9 and 22, the association of a deletion of a portion of 13q14 with retinoblastoma and the association of a translocation involving chromosomes 8 and 14 with Burkitts lymphoma. Current progress in elucidating new tumor specific abnormalities is limited by the difficulty of producing representative, high quality banded metaphase spreads for cytogenetic analysis. These problems stem from the fact that many human tumors are difficult or impossible to grow in culture. Thus, obtaining mitotic cells is usually difficult. Even if the cells can be grown in culture, there is the significant risk that the cells that do grow may not be representative of the tumorigenic population. That difficulty also impedes the application of existing genetic knowledge to clinical diagnosis and prognosis.

The present invention overcomes these limitations by allowing detection of specific structural and numerical aberrations in interphase nuclei. These aberrations are detected as described supra. Hybridization with whole chromosome probes will facilitate identification of previously unknown aberrations thereby allowing rapid development of new associations between aberrations and disease phenotypes. As the genetic nature of specific malignancies becomes increasingly well known, the interphase assays can be made increasingly specific by selecting hybridization probes targeted to the genetic lesion. Translocations at specific sites on selected chromosomes can be detected by using hybridization probes that closely flank the breakpoints. Use of these probes allows diagnosis of these specific disease phenotypes. Translocations may be detected in interphase because they bring together hybridization domains that are normally separated, or because they separate a hybridization domain into two, well separated domains. In addition, they may be used to follow the reduction and reemergence of the malignant cells during the course of therapy. Interphase analysis is particularly important in such a application because of the small number of cells that may be present and because they may be difficult or impossible to stimulate into mitosis.

Duplications and deletions, processes involved in gene amplification and loss of heterozygosity, can also be detected in metaphase spreads and interphase nuclei using the techniques of this invention. Such processes are implicated in an increasing number of different tumors.

## VI. Detection of the Retinoblastoma Gene in Metaphase Chromosomes and Interphase Nuclei

### Probes.

Fourteen lambda phage clones which form three contigs (overlapping nucleic acid sequences) and span the exons of the Rb-1 gene were obtained from Yuen Kai Fung [Division of Hematology and Oncology, Childrens' Hospital of Los Angeles, University of Southern California, Los Angeles, CA 90027 (USA)]. The phage DNA was labeled either by biotin-dUTP or digoxigenin-dUTP using the Bio Nick™ Labeling System [BRL Life Technologies, Inc., Gaithersburg, MD (USA)].

A probe specific to the 13/21 centromeric alphoid repetitive sequence was used in assisting identification of chromosome 13 in metaphase preparations. The 13/21 centromeric probe was prepared by the polymerase chain reaction (PCR) according to methods detailed in Weier et al., Hum. Genet., 87(4): 489-494 (1991). Briefly, the probe was made by PCR using flow sorted human chromosome 21s as a template and two primers (30 uM) specific for the alphoid sequence. The product was labeled during the PCR reaction by including biotin 11-dUTP (100%). Oligonucleotide primers used were W21R1 (5′-GGATAGCTTAACGATTTCGTTGGAAAC-3′) and W21R2 (5′-CAAACGTGCTCAAAGTAAGGGAATG-3′). They were synthesized using phosphoramidite chemistry on a DNA synthesizer [Applied Biophysics, Foster City, CA (USA) model 380B]. Synthesis and further purification of the oligonucleotides by C18 reverse phase chromatography and HPLC was performed according to the specifications of the manufacturer (Waters Chromatography, Milford, MA (USA)]. Using the flow sorted chromosomal DNA as a template these primers generate a 135 bp product.

### Cell samples.

PHA-stimulated normal peripheral blood lymphocytes, cultured human skin fibroblasts, two fibroblast cell lines from retinoblastoma patients GM05877 46, XX, del(13) (pter-q14.1::q21.2-qter) and GM01152A 46, XX, del(13) (pter-q14.1::q22.1-qter) obtained from the NIGMS (National Institute of General Medical Science) Human Genetic Mutant Cell Repository [Camden, NJ (USA)], and clinical human breast cancer samples obtained either by fine-needle aspiration or disaggregation of fresh tumor tissue were used [made available by Fred Waldman, M.D., Department of Laboratory Medicine, University of California, San Francisco, CA (USA)]. Cell lines were either treated with colcemid to prepare metaphases or grown to confluency to obtain G0/G1 interphase nuclei. All samples were fixed in 3:1 methanol-acidic acid and dropped on microscope slides. Before in situ hybridization, the slides were treated with (1 ug/50 ml) Proteinase K [Boehringer Mannheim GmbH, Indianapolis, IN (USA)] for 7.5 min at 37°C.

In situ hybbridization.

FISH was done using modifications of previously published methods [Pinkel et al., PNAS (USA), 83: 2934 (1986); Trask et al., Genomics, 5: 710 (1989)]. The hybridization mixture consisting of 20-40 ng of labeled probe, 5-10 ug of unlabeled human placental DNA in 50% formamide, 2XSSC and 10% dextran sulphate was denatured for 5 min at 70°C and then allowed to reanneal for 30-60 min at 37°C. In dual color hybridizations, 20 ng of Rb 3' end probe and 20 ng of Rb 5' end probe was used. In cohybridizations with the 13/21 centromeric probe, 2 ng of the centromeric probe was used with 20-40 ng of the Rb-1 probe. The slides were denatured in 70% formamide, 2XSSC at 70°C for 2 min. Hybridization was done under a coverslip in a moist chamber at 37°C for 2 days.

Staining.

Briefly, the slides were washed three times in 50% formamide, 2XSSC for 10 min, twice in 2XSSC and once in 0.1XSSC at 45°C. Biotin-labeled specimens were stained with (5 ug/ml) FITC- or Texas Red-Avidin [Vector Laboratories, Inc., Burlingame, CA (USA)] in 4XSSC/1% BSA for 30 minutes at room temperature. Anti-avidin (Vector Laboratories, Inc.) (5 ug/ml) incubation was done in PNM buffer for 20 min followed by a second layer of FITC/Texas Red-Avidin in PNM buffer. Digoxigenin-labeled probes were detected using an FITC-labeled sheep antibody against digoxigenin and a second layer of rabbit anti-sheep FITC antibody (Vector Laboratories, Inc.). Before each antibody or avidin treatment, the slides were preblocked with either 1%BSA or PNM. Between the antibody incubations, the slides were washed twice in 4XSSC and once in 4XSSC/0.1% TRITON X-100 or three times in PN buffer. Nuclei were counter-stained with 0.2 ug/ml propidium iodide or 0.27 uM DAPI in an antifade solution.

Fluorescence microscopy and digital image analysis.

A Nikon fluorescence microscope was used in most of the analyses. For interphase analysis at least 150 nuclei were scored from each sample. To map the metaphase hybridization signals accurately, digital image analysis was used. [Zeiss Axioplan; Quantex Corporation (Sunnyvale, CA) (USA)] The multicolor images were stored on computer magnetic disks at an approximate resolution of 19 pixels/um and analyzed using a specific software program based on the TCL-Image software package. The program defines the contour of the DAPI-stained chromosome and draws the longitudinal axis of the chromosome. The hybridization signals are then overlaid in pseudocolors on the chromosome image to calculate their relative position in terms of the distance from the p-telomere compared to the total chromosome length (=fractional length scale).

Results.

Using fourteen lambda phage clones together (Rb-1 probe), a bright and specific hybridization signal on lymphocyte metaphase preparations in the mid-region of the q-area of chromosome 13 were obtained (Figs 1A and 1B). A more accurate localization of the Rb-1 gene was achieved by digital image analysis. The mean distance (+S.D.) of the Rb-1 signal from the 13 pter (p-termines) was determined to be compatible with the location of the Rb-1 gene in the band q14.2 (Fig. 1C). Analysis of the Rb-1 hybridization from interphase nuclei was first attempted in normal lymphocytes and fibroblasts (Fig. 1D). Two hybridization signals representing the two gene alleles were detected in about 90% of the nuclei (Fig. 1A). The remaining 10% showed either one or three fluorescence signals. In interphase, the fluorescence signal was not always singular but could appear as 2-4 small adjacent individual spots probably because the Rb-1 probe consists of three separate contigs.

Two fibroblast cell lines from retinoblastoma patients with homozygous deletions affecting the Rb-1 region were used to test the sensitivity of FISH in detecting deletions as an absence of a hybridization signal. In both cell lines one Rb-1 signal was detected in about 70-80% of the interphase nuclei (Figs. 1G and 2B). Metaphase preparations from those cell lines hybridized simultaneously with the Rb-1 and the 13/21 centromeric probes showing that the normal chromosome 13 had both the centromeric and the Rb-1 signals, whereas the other slightly shortened chromosome 13 hybridized only with the centromeric probe (Fig. 1E and 1F).

The Rb-1 probe was also used to study fine needle aspirations and touch preparations from different breast cancer patients. Although the breast cancer samples had more non-specific background fluorescence than cultured cells, it was still possible to evaluate Rb-1 gene copy numbers from individual tumor nuclei (Fig. 1H). As

shown in Table 2 below, marked genetic heterogeneity both within and between breast tumors was found in the analysis of six cases. The modal Rb-1 gene copy number varied from 1-3 in the tumors. As compared to experiments with cell cultures, the clinical samples showed a higher percentage of cells without any Rb-1 signals. Table 2 shows the percentage of nuclei exhibiting a defined number of Rb-1 signals/nucleus in six clinical breast cancer specimens. The results represent the mean of 2-3 hybridization experiments. At least 150 cells were scored from each slide.

## Table 2

| Tumor | No. signals/nuclei (%) | | | | | | | DI |
|-------|---|---|---|---|---|---|---|------|
|       | 0 | 1 | 2 | 3 | 4 | 5 | 6 |      |
| B156  | 22 | 23 | 45 | 5 | 5 | 0 | 0 | 1.50 |
| B245  | 28 | 36 | 31 | 3 | 2 | 0 | 0 | 1.35 |
| B249  | 2 | 10 | 23 | 36 | 25 | 3 | 0.5 | 1.82 |
| B252  | 21 | 14 | 43 | 14 | 3 | 3 | 1 | 1.87 |
| B259  | 36 | 49 | 12 | 2 | 1 | 0 | 0 | 1.64 |
| B236  | 16 | 3 | 11 | 33 | 20 | 10 | 6 | 2.25 |

DI = DNA index

To detect subregions of the Rb-1 gene, single phage clones spanning only 8-20 kb of the 200 kb Rb-1 gene were used as hybridization probes. The hybridization signals from such probes could be seen both in metaphase chromosomes and interphase nuclei, but the hybridization efficiency was significantly less than with the pooled Rb-1 probe. In contrast, if 2-5 contiguous phage clones were pooled, the hybridization was more efficient and more easily evaluated. This approach was used to visualize the 3' and 5' ends of the Rb-1 gene in interphase nuclei with differently labeled probes in a dual-color hybridization (Fig. 1I).

Thus, in conclusion, the Rb-1 gene was mapped 13q14 by FISH and digital image analysis, confirming the location of the gene to be in close proximity to the esterase D locus [Sparkes et al., Science, 208: 1042-1044 (1980)]. Also shown in this section is that the methods of this invention can be used to detect deletions involving the Rb-1 locus. In order to verify the presence of a deletion from unbanded propidium iodide stained metaphase preparations, it was necessary to use a reference probe which in the representative example of this section was a 13/21 pericentromeric alpha satellite probe. Chromosome 13s with a deletion in the Rb-1 locus were thereby identified.

The representative examples of this section demonstrate that chromosome-specific painting can be used to detect Rb-1 gene deletions from interphase nuclei of cultured fibroblasts from retinoblastoma patients known to have a constitutive deletion in 13q. The usefulness of chromosome-specific staining is determined by the hybridization efficiency obtained, which in turn experentially has been found to be dependent on probe target size. Previous studies on interphase FISH have mainly been done using probes to pericentromeric repetitive sequences with a target size of a few megabases with hybridization efficiencies around 90-95% (Pinkel et al., supra 1986). In experiments using the 150 kb Rb-1 probe with cultured cells, the hybridization efficiency obtained in interphase was about 80-90%, whereas in clinical samples the efficiency was apparently less since a number of cells exhibited no hybridization signals. Poor hybridization efficiency might therefore lead to misinterpretation of a deletion. Further, in solid tumors having very complex karyotypic abnormalities, the distinction between numerical chromosome aberrations and structural abnormalities may be difficult to evaluate. Therefore, it is preferred in analyzing large numbers of solid tumors to co-hybridize with other reference probes for the same chromosome to control for the hybridization efficiency as well as for the presence of numerical chromosomal abnormalities. The centromeric 13/21 alpha satellite probe used successfully in metaphase preparations cannot be applied to interphase analysis because the signals for chromosomes 13 and 21 cannot be distinguished. Therefore, for interphase analysis, it is preferred that a reference probe specific for chromosome 13 be used.

The studies on clinical breast cancer material described in this section demonstrate the genetic heterogeneity of breast cancer. The evaluation of this heterogeneity coupled with the possibility of studying gene copy numbers from morphologically defined individual tumor cells are major advantages of the chromosome-specific staining methods of this invention.

VII. Detection of Chromosome 3 and 17 Aberrations Associated with Cancer

Probes.

Two centromeric-specific alpha satellite probes are used in the representative examples of this section; one is specific to chromosome 17, and the other to chromosome 3. The centromeric-specific probes were prepared similarly as the 13/21 specific centromeric probes were, as indicated above in Section VI. Specifically, those probes were prepared using a polymerase chain reaction (PCR) process employing a thermostable enzyme [Saiki et al., Science, 239: 487-491 (1988)] as follows.

Probe specific for alpha satellite centromeric repeats on human chromosome 17.

Approximately 50 ng (nanograms) of DNA isolated from the Bluescribe plasmid library for chromosome 17 (pBS17) were used as the DNA template. Pinkel et al., PNAS USA, 85: 9138-9142 (Dec. 1988) describes the preparation of such Bluescribe libraries as subcloning an entire chromosome 17 library, which is publicly available, for example, as LL17NS01 or LA17NS03 [Van Dilla et al., Bio/Technology, 4: 537-552 (June 1986)] into Bluescribe plasmids [Statagene, La Jolla, CA (USA)].

The reaction buffer consisted of 5 units of Thermus aquaticus (tag) DNA polymerase [Bethesda Research Laboratories, Gaithersburg, MD (USA)]; mixed with 100 ul amplification/biotinylation buffer [10 mM Tris-HC1, pH 8.4 at 20°C; 1.5 mM MgCl$_2$; 5 mM KC1; and 0.2 mM each of 2'-deoxyadenosine 5'-triphosphate (dATP), 2'-deoxyguanosine 5'-triphosphate (dGTP), and biotin-II-dUTP [all the deoxynucleotide triphosphates were from Sigma, St. Louis, MO (USA)]; and 1.2 uM each of the two primers WA1 and WA2 [WA1 5'-GAAGCT-TA(A/T'(C/G)T(C/A)ACAGAGTT(G/T)AA-3' and WA2 5'-GCTGCAGATC(A/C)C(A/C)AAG(A/T/C)AGTTTC-3']. Mineral oil (100 ul) [Squibb, Princeton, NJ (USA)] was layered on top of the reaction mixture to prevent evaporation during the PCR.

DNA amplification and simultaneous biotinylation was performed during 45 cycles using an automated thermal cycling system [Weier and Gray, DNA, 7: 441-447 (1988)]. Each cycle began with a thermal denaturation step of 90 seconds at 94°C (120 seconds for the initial denaturation). Primer annealing during the second step of each cycle was performed at 53°C for 90 seconds. The temperature was then increased slowly (7°C/minute) to 72°C. The cycle was completed by holding that temperature for 120 seconds for primer extension. Amplification of alpha satellite DNA was confirmed visually by electrophoresis of 5 ul aliquots of the PCR reaction mixture on 4% agarose gels (BRL) in 40 mM Tris-acetate, 1 mM EDTA, pH 8.0 containing 0.5 ug/ml ethidium bromide [Maniatis et al., Molecular Cloning: A Laboratory Manual (Cold Spring Harbor Laboratory, Cold Spring Harbor, N.Y.) (USA) (1986)]. The concentration of double stranded DNA in the reaction was determined to be 229 ug/ml by Hoeschst 33258 fluorescence using a TK 100 fluorometer [Hoefer Scientific, San Francisco, CA (USA)].

Probe specific for alpha satellite centromeric repeats on human chromosome 3.

In vitro DNA amplification was performed using approximately 80 ng of CsC1 gradient isolated DNA from the Bluescribe plasmid library for chromosome 3 (pBS3) (400 ng/ul) as amplification template per 200 ul reaction mixture. The reaction buffer was the same as that used to prepare the chromosome 17 centromeric-specific probe above except that dTTP is used instead of Biotin-11-dUTP.

PCR was performed for 30 cycles using an automated thermal cycler [Perkin-Elmer/Cetus, Norwalk, CT (USA)]. The DNA template was denatured at 94°C for 1 minute (1 minute 30 seconds during the first cycle). Primer annealing and extension were performed at 53°C and 72°C, respectively. Probe biotinylation and further amplification was accomplished in a second reaction by adding a 5 ul aliquot of the product to 200 ul reaction mix containing 0.25 mM Biotin-11-dUTP [Sigma St. Louis, MO (USA)] in the absence of dTTP and 10 units of Taq polymerase [Weier et al., J. Histochem. Cytochem., 38: 421-426 (1990)]. The amplification/biotinylation reaction was performed during an additional 20 PCR cycles. Amplification of degenerate alpha satellite DNA was confirmed visually by gel electrophoresis of 10 ul aliquots of the PCR reaction in either 1.8% or 4% agarose (BRL) in 40 mM Tris-acetate, 1 mM EDTA buffer, pH 8.0 containing 0.5 ug/ml ethidium bromide. After completion of PCR, labeled probe and amplified DNA were stored at -18°C.

Chromosome 3 alpha satellite centromeric-specific repetitive probe.

Another chromosome 3 centromeric-specific probe called palpha 3-5 was obtained from Huntington Willard, Ph.D. [Department of Genetics, Stanford University School of Medicine, Stanford, CA (USA)]. That probe was described at the Ninth International Workshop on Human Gene Mapping in Paris (Cytogenet. Cell Genet., 46 (1-4): 424, 564 and 712 (1987), and 51 (1-4): 111 (1989)]; and a similar probe is described in Waye and Willard, Chromosoma (Berl), 97: 475-480 (1989). The palpha 3-5 probe was labeled with AAF according to conventional methodology for use in experiments described below.

3p cosmid probe.

A 3p cosmid probe called cCl3-787 was obtained from Yusuke Nakamura, MD, Ph.D. [Division of Biochemistry, Cancer Institute, Toshima, Tokyo, 170, Japan]. Its isolation and mapping to 3p21.2-p21.1 is described in Yamakawa et al., Genomics, 9(3): 536-543 (1991). That probe was amplified and labeled with biotin according to a PCR linker/adapter method described in Johnson, Genomics, 6: 243-251 (1990) and Saunders et al., Nuc. Acids. Res., 17 (22): 9027-9037 (1989).

3q cosmid probe.

A 3q cosmid probe named J14R1A12 was developed and provided by Wen-Lin Kuo [Biomedical Department, P.O. Box 5507 (L-452), Lawrence Livermore National Laboratory, Livermore, CA 94550 (USA)]. It was obtained from a chromosome 21 flow sorted library prepared by conventional means. It was mapped to the location 3q26 using an extrapolation of the fractional length for the probe to a chromosome 3 ideogram. It was labeled with biotin-dUTP using the Bio Nick™ Labeling System [BRL Life Technologies, Inc. Gaithersburg, MD (USA)].

Composite Whole Chromosome 3-Specific Probe.

A probe specific for the whole chromosome 3 was a Bluescribe plasmid library for chromosome 3 prepared according to Pinkel et al., PNAS (USA), 85: 9138-9142 (Dec. 1988) and named pBS3.

Cell Samples.

Used in the following examples were PHA-stimulated normal peripheral blood lymphocytes; two ovarian cancer cell lines, designated as RMUG-S and RMUG-L, provided by Shiro Nozawa, MD, Ph.D. (Department of Obstetrics/Gynecology, School of Medicine, Keio University, 35 Shinano-machi, Shinjuku-ku, Tokyo, 160, Japan) and described in Sakayori et al., Human Cell, 3(1): 52-56 (1990); and a uterine cervical adenocarcinoma cell line, named TMCC-1, described in Sakamoto, J. Tokyo Med. College, 46(5): 925-936 (1988). RMUG-S is a hypodiploid cancer cell line, whereas RMUG-L is a hypertriploid cancer cell line. Both lines were cloned from the same clinical specimen.

In situ hybridization and staining.

The protocols resulting in the hybridizations shown in Figures 3, 4 and 5 were the same as those used in Section VI except that for Figure 3, the hybridization mixture contained 5 ug of herring sperm DNA instead of 5 ug of unlabeled placental DNA, and reannealing of the denatured probe just before in situ hybridization was not performed; and for Figure 4, the hybridization mixture contained a reduced amount of unlabeled human placental DNA explicitly 0.5 ug.

For the results shown in Figures 6 and 7, the hybridization protocols differed from that detailed in Section VI in that before hybridization, the slides were pretreated with 100 ug/ml of RNase for 30 minutes at 37°C and then treated with 1 ug/ml of Proteinase K for 7.5 minutes at 37°C. The hybridization mixture comprised 1 ul biotinylated 3p cosmid (wherein the PCR product was diluted 1:10 with double distilled, deionized water) or 30 ng - 40 ng 3q cosmid; 2 ng chromosome 3 alpha satellite centromeric-specific probe (palpha 3-5) labeled with AAF; 5 ug - 10 ug unlabeled human placental DNA; and 7.0 ul of master mix [which consists of 5 ml formamide to which 1 g dextran sulphate and 1 ml 20XSSC (prepared using deionized, double distilled water) was added, the pH of which was adjusted to 7.0 with 1N HC1 and the final volume to 7 ml was completed with deionized, double distilled water. The master mixture is stored at -20°C indefinitely.

For the results shown in Figures 6 and 7, a dual color staining protocol was performed essentially according to Trask and Pinkel, Methods in Cell Biology, 33: 383-400 (1991). Briefly, the slides were washed three times in the washing solutions for 10 minutes each at 45°C, 2XSSC for 10 minutes at 45°C, 0.1XSSC for 10 minutes, and PN buffer (wherein the percentage of NP-40 is 0.05% rather than 0.1%) for 5 minutes at room temperature.

The washing solutions comprise 50% formamide:2xSSC (75 ml formamide; 15 ml 20xSSC; and 60 ml deionized, double distilled water wherein the pH is adjusted to 7.0 with 1 N HC1).

The slides were preblocked with 20 ul PNM buffer under a 22 x 22 mm coverslip at room temperature for 5 minutes inside a dark moist chamber. The coverslip was then taken off, and the PNM buffer drained from the slide.

Twenty ul of anti-AAF and Avidin-Texas Red solution were added to the cells' area per slide and then covered with a 22 x 22 mm coverslip. The slides were incubated within a dark moist chamber for 1 hour at room temperature. The anti-AAF and Avidin-Texas Red solution was prepared by adding 8 ul of 0.25 ug/ul Avidin-Texas Red (Vector Laboratories, Inc.) to 1 ml of the supernatant of anti-AAF producing mouse cells.

The coverslips were then removed, and the slides washed with intermittent shaking in the PN buffer thrice for 10 minutes each in a dark place.

The cells were then preblocked as described above. Twenty ul of goat-anti-mouse-FITC antibody and biotinylated anti-Avidin antibody solution was added to the cells' area on each slide. The cells were covered with a coverslip and incubated inside a dark moist chamber for one hour at room temperature. The antibody solution per ml comprised 20 ul of goat-anti-mouse FITC antibody (from Cal tag [Burlingame, CA (USA)], i.e., the final concentration is 20 ug/ml) and 10 ul biotinylated anti-avidin antibody solution at the concentration of 0.5 mg/ml (from Vector Laboratories, Inc., i.e., the final concentration is 5 ug/ml) to 970 ul antibody dilution buffer [IX Dulbecco's PBS (Ca, Mg free), 0.05% TWEEN 20, 2% normal goat serum].

The coverslips were removed from the slides, and the slides are then washed with intermittent shaking in the PN buffer thrice for 10 minutes each in a dark place. The cells were then preblocked as indicated above.

Twenty ul of the Avidin-Texas Red solution were added to the cells' area per slide, and then a coverslip was applied. The slides were then incubated inside a dark moist chamber for one hour at room temperature. The Avidin-Texas Red solution comprises 8 ul of a 0.25 ug/ul Avidin-Texas Red (Vector Laboratories, Inc.) to 1 ml of the antibody dilution buffer.

The coverslips were then removed from the slides, and the slides washed in the PN buffer thrice for 10 minutes each in a dark place. About 8 ul of 0.8 um DAPI (counterstain) in an anti-fade solution is prepared according to Johnson and Nogueria, J. Immunol. Methods, 43: 349 (1981) [100 mg p-phenylene-diamine dihydrochloride (Sigma P1519) in 10 ml Dulbecco's PBS; pH adjusted to 8 with 0.5 M carbonate-bicarbonate buffer; 90 ml glycerol added; filtered through 0.22 um; stored at -20°C] was added to the slides. The slides mounted with the anti-fade solution can be stored in a dark chamber at 4°C.

Results.

Figure 3A shows the hybridization of the chromosome 17 centromeric-specific alpha satellite probe to normal lymphocytes wherein in metaphase chromosomes, two bright signals are seen, and in interphase nuclei, two bright, tight hybridization domains are visible. Figure 3B shows the hybridization of that probe to the human ovarian mucinous cysto-adenocarcinoma (RMUG-L), wherein in both metaphase and interphase, four signals are visible.

These examples are representative of the use of chromosome-specific repeat probes for the detection of numerical chromosome aberrations on chromosome 17 which are used as a component of the high complexity staining probes of this invention.

Figures 4A and B show hybridization of the whole chromosome composite probe for chromosome 3 (pBS3) (A) to normal lymphocytes and (B) to the ovarian cancer cell line (RMUG-L). Two normal chromosome 3s are seen in Figure 4A, whereas four chromosome 3s are seen in the ovarian cancer cell line (Figure 4B), of which two are apparently shorter than the intact chromosome 3s, a pattern which is congruent with a 3p deletion in the karyotype.

Chromosome-specific recombinant lambda libraries have been constructed for all the human chromosomes by the National Laboratory Gene Library Project [Van Dilla et al. (1986), supra]. Subsequently, those libraries were subcloned into Bluescribe plasmid vectors (Stratagene), and whole chromosome composite probes were generated from the DNA extracted from those plasmids [Fuscoe et al., Genomics, 5: 100-109 (1989); Collins et al., Genomics 11(4): 997-1006 (1991)]. Staining with such whole chromosome composite probes can be used to detect not only large deletions but also subtle translocations and to identify the origin of marker chromosomes.

Figures 5A and B as well as Figure 1 provide examples of the use of locus-specific probes to count the copy number of specific genes in tumor cells and to detect changes in patterns of hybridization domains. Figures 5A and B provide representative examples of the use of locus-specific probes to detect translocations. As indicated above, such examples are the first step in locating exact information on genetic rearrangements within a locus. The 3q cosmid probe employed in these studies is just one of many potential probes from chromosome 3 that can be used [Yamakawa et al., Genomics 9(3): 536-543 (1991)]. Preferably, in metaphase spreads, probes with different labels according to their order in a normal chromosome 3 may be used to detect any structural chromosomal aberrations differing from the standard. Further, in either metaphase spreads or interphase nuclei, probes with different labels according to their location in normal chromosomes may be used to detect structural chromosomal aberrations, for example, commonly deleted lesions found in cancer cells.

Figures 5A and B show the hybridization of a chromosome 3 centromeric-specific alpha satellite repeat probe (the one generated by the PCR process with the primers WA1 and WA2) and a 3q cosmid probe (J14R1A12 mapped to 3q26) to, respectively (A) normal lymphocytes and (B) a uterine cervical adenocarcinoma cell line (TMCC-1). As indicated in the description of the figures above, two pairs of normal chromosome 3s are illustrated in (A) whereas a pair of cosmid signals specific to chromosome region 3q was found to be translocated to another chromosome.

Figures 6A and 6B show dual color hybridizations to normal lymphocytes, metaphase spread and interphase nucleus, respectively. Figures 7A and 7B show comparable hybridizations to an ovarian cancer cell line. A chromosome 3 centromeric-specific repetitive probe (AAF labeled palpha 3-5 from Huntington Willard) and 3p region-specific (3p21.2 p21.1) cosmid probe (cCI3-787) (that is biotinylated and amplified and labeled by linker adapter PCR) were employed in such hybridizations. Two hundred interphase nuclei were scored for each experiment.

In Figure 6A, the image of chromosome 3 from normal lymphocytes was digitized by the digital fluorescent microscope and shows that one chromosome 3 centromeric-specific green signal and one chromosome 3p region-specific red signal for each chromatid were visible.

In Figure 6B, the picture of an interphase nucleus from normal lymphocytes, taken with a conventional fluorescent microscope, shows two greenish hybridization domains for the centromeric specific probes and two reddish domains for the 3p probe. It was commonly observed that a pair of cosmid signals on both chromatids of one chromosome 3 fuses into a single spot in interphase nuclei.

Figure 7A shows a partial metaphase spread, one chromosome 3 shows a normal hybridization pattern whereas the other shows a 3p deletion. Figure 7B shows in interphase nuclei, four large greenish domains for the centromeric probe and two small reddish hybridization domains for the 3p probe, indicating aneuploidy of chromosome 3, wherein two out of four of the chromosome 3s have a 3p deletion.

Eighty-six percent of the interphase nuclei of the normal lymphocytes showed a normal pattern of two green centromeric signals and two red signals of the 3p cosmid probe. However 98% (94%) of interphase nuclei of the RMUG-S (RMUG-L) cells showed a lesser number of red signals (3p cosmid) than green signals (centromere) suggesting a chromosome 3p deletion in those cell lines. Among those nuclei, 53% (52%) of interphase nuclei of the RMUGS-S (RMUG-L) cells showed two domains of the 3p cosmid signal and 4 domains of the chromsome 3 centromeric-specific signals.

Figures 8 and 9 show the results of simultaneous hybridizations of an AAF-labeled chromosome 3 centromeric-specific probe (from H. Willard) and a biotinylated chromosome 3q cosmid probe (J14R1A12) wherein in Figure 8 the target is a metaphase spread and interphase nucleus of normal lymphocytes and wherein in Figure 9 the target is an interphase nucleus from the ovarian cancer cell line (RMUG-S). A pattern for a normal chromosomal complement is shown in Figure 8 as two chromosome 3 centromeric-specific green signals and two pairs of chromosome 3q cosmid red signals per cell. An abnormal pattern is shown in Figure 9 as four chromosome 3 centromere-specific green signals and four chromosome 3q cosmid red signals, indicating that the cell contains four long arms of chromosome 3. The results shown in Figures 8 and 9 support the feasibility of detecting 3p deletions in interphase nuclei of tumor cells if combined with the findings of domain number for 3p cosmid signals. Such a methodology can be applied to detect 3p deletions in clinical tumor specimens for basic research on tumorigenesis and progression, and adjunctive diagnosis of cancers associated with 3p deletions, such as, small cell lung cancer, renal cell cancer and ovarian cancer.

The descriptions of the foregoing embodiments of the invention have been presented for purpose of illustration and description. They are not intended to be exhaustive or to limit the invention to the precise form disclosed, and obviously many modifications and variations are possible in light of the above teachings. The embodiments were chosen and described in order to best explain the principles of the invention and its practical application to thereby enable others skilled in the art to best utilize the invention in various embodiments and with various modifications as are suited to the particular use contemplated. All references cited herein are hereby incorporated by reference.

## Claims

1. A method of staining targeted chromosomal material based upon nucleic acid sequence employing high complexity nucleic acid probes wherein the staining pattern produced is indicative of the presence or absence of one or more genetic rearrangements involving chromsomes 3, 13 and/or chromosome 17.

2. A method according to Claim 1 wherein the one or more genetic rearrangements is or are associated with cancer, and the targeted chromosomal material is one or more metaphase and/or interphase chromosomes, or one or more regions thereof.

3. A method according to Claim 1 wherein said one or more genetic rearrangements is or are selected from the group consisting of translocations, inversions, insertions, aneuploidy, amplifications and deletions.

4. A method according to Claim 2 wherein the cancer is selected from the group consisting of retinoblastomas, osteosarcomas, lung cancer, breast cancer, renal cell cancer, ovarian cancer and/or uterine cancer.

5. A method according to Claim 4 wherein the genetic rearrangement is a 13q deletion within the retinoblastoma tumor suppressor gene, and the cancer is selected from the group consisting of retinoblastomas, osteosarcomas, small cell lung cancer and breast cancer.

6. A method according to any one of the preceding claims wherein one component of the hight complexity nucleic acid probes is a reference probe.

7. A method according to Claim 6 wherein the reference probe is a centromeric-specific alpha satellite probe.

8. A method according to Claim 4 wherein the genetic rearrangement is a 3p deletion, and the associated cancer is selected from the group consisting of small cell lung cancer, renal cell cancer, uterine cancer and ovarian cancer.

9. High complexity nucleic acid probes for the detection of one or more genetic rearrangements associated with the retinoblastoma tumor suppressor gene, chromosome 3, and/or chromosome 17 in humans.

10. A method of detecting genetic rearrangements associated with the retinoblastoma tumor suppressor gene, chromosome 3, and/or chromosome 17 in humans comprising the steps of:
    a. hybridizing the probes of Claim 9 to targeted chromosomal material;
    b. observing and/or measuring the proximity of and/or other characteristics of the signals from said probes; and
    c. determining from said observations and/or measurements obtained in step b) whether one or more genetic rearrangements has or have occurred.

11. A chromosome-specific staining reagent that provides staining patterns indicative of one or more genetic rearrangements associated with the retinoblastoma tumor suppressor gene, chromosome 3, and/or chromosome 17 in humans, produced by the process of:
    isolating chromosome-specific DNA selected from the group consisting of chromosomes 3, 13 and/or 17;
    amplifying pieces of the isolated chromosome-specific DNA;
    disabling the hybridization capacity of and/or removing shared repetitive sequences contained in the amplified pieces of the isolated DNA to form a collection of nucleic acid fragments which hybridize predominantly to targeted chromosomal DNA on chromosomes 3, 13 and 17; and
    labeling the nucleic acid fragments of the collection to form a heterogeneous mixture of nucleic acid fragments.

12. A chromosome-specific staining reagent according to Claim 11 wherein said step of amplifying said pieces of isolated DNA is performed by using the polymerase chain reaction (PCR).

13. A method according to ant one of claims 1 to 8 or 10 performed in conjunction with digital image analysis.

14. A method of staining targeted chromosomal material based upon nucleic acid sequence employing high complexity nucleic acid probes wherein the staining pattern produced is indicative of one or more genetic rearragements associated with a tumor suppressor gene.

15. A method according to Claim 14 wherein the tumor suppressor gene is either the retinoblastoma tumor suppressor gene or located on the p arm of chromosome 3, and the genetic rearrangement is a deletion.

FIG. 1A

FIG. 1B

FIG. 1C

FIG. 1D

FIG.1E

FIG.1F

FIG.1G

FIG.1H

**FIG. 1I**

**FIG. 2A**

**FIG. 2B**

FIG. 3A

FIG. 3B

FIG. 4A

FIG. 4B

FIG.5A

FIG.5B

FIG.6A

FIG.6B

FIG. 7A

FIG. 7B

FIG. 8

FIG. 9